# EUROPEAN PATENT SPECIFICATION

(11) **EP 1 746 172 B1**
(45) Date of publication and mention of the grant of the patent: **05.10.2016**
(21) Application number: 06013329.5
(22) Date of filing: 28.06.2006
(51) Int. Cl.: C12Q 1/68, C12Q 1/70

(54) **Asymmetric PCR coupled with post-PCR characterization for the identification of nucleic acids**
Asymmetrische PCR verbunden mit post-PCR Charakterisierung zur Identifizierung von Nukleinsäuren
PCR asymétrique couplée à une caractérisation post-PCR pour l'identification d' acides nucléiques

(30) Priority: 30.06.2005 US 696293 P; 30.06.2005 US 695991 P; 30.06.2005 US 696303 P; 30.06.2005 US 696253 P
(43) Date of publication of application: 24.01.2007
(73) Proprietor: Roche Diagnostics GmbH, 68305 Mannheim (DE); F. Hoffmann-La Roche AG, 4070 Basel (CH)
(72) Inventor: Newton, Nicolas, Oakland, CA 94619 (US); Will, Stephen Gordon, Oakland, CA 94611 (US)

(56) References cited:
- WO-A2-01/48237
- LIN ZHILI ET AL: "A high throughput beta-globin genotyping method by multiplexed melting temperature analysis." MOLECULAR GENETICS AND METABOLISM, vol. 81, no. 3, March 2004 (2004-03), pages 237-243, XP002417472 ISSN: 1096-7192
- MACKAY I M ET AL: "Molecular assays for detection of human metapneumovirus" JOURNAL OF CLINICAL MICROBIOLOGY 01 JAN 2003 UNITED STATES, vol. 41, no. 1, 1 January 2003 (2003-01-01), pages 100-105, XP002417473 ISSN: 0095-1137
- SCHROTER M ET AL: "Genotyping of hepatitis C virus types 1, 2, 3, and 4 by a one-step LightCycler method using three different pairs of hybridization probes" JOURNAL OF CLINICAL MICROBIOLOGY 2002 UNITED STATES, vol. 40, no. 6, 2002, pages 2046-2050, XP002414434 ISSN: 0095-1137
- BULLOCK GRANT C ET AL: "Hepatitis C genotype determination by melting curve analysis with a single set of fluorescence resonance energy transfer probes." CLINICAL CHEMISTRY, vol. 48, no. 12, December 2002 (2002-12), pages 2147-2154, XP002414623 ISSN: 0009-9147
- STUYVER LIEVEN ET AL: "Second-generation line probe assay for hepatitis C virus genotyping" JOURNAL OF CLINICAL MICROBIOLOGY, vol. 34, no. 9, 1996, pages 2259-2266, XP002414344 ISSN: 0095-1137
- SANCHEZ J AQUILES ET AL: "Linear-After-The-Exponential (LATE)-PCR: An advanced method of asymmetric PCR and its uses in quantitative real-time analysis." PROCEEDINGS OF THE NATIONAL ACADEMY OF SCIENCES OF THE UNITED STATES OF AMERICA, vol. 101, no. 7, 17 February 2004 (2004-02-17), pages 1933-1938, XP002417485 ISSN: 0027-8424
- LOGAN J M J ET AL: "Rapid identification of Campylobacter spp. by melting peak analysis of biprobes in real-time PCR" IJMM INTERNATIONAL JOURNAL OF MEDICAL MICROBIOLOGY, vol. 291, no. Supplement 31, September 2001 (2001-09), pages 106-107, XP002417504 & 11TH INTERNATIONAL WORKSHOP ON CAMPYLOBACTER, HELICOBACTER AND RELATED ORGANISMS; FREIBURG, GERMANY; SEPTEMBER 01-05, 2001 ISSN: 1438-4221
- CHAN S-W ET AL: "ANALYSIS OF A NEW HEPATITIS C VIRUS TYPE AND ITS PHYLOGENETIC RELATIONSHIP TO EXISTING VARIANTS" JOURNAL OF GENERAL VIROLOGY, vol. 73, no. 5, 1992, pages 1131-1141, XP000378699 ISSN: 0022-1317
- J. A. Sanchez ET AL: "Linear-After-The-Exponential (LATE)-PCR: An advanced method of asymmetric PCR and its uses in quantitative real-time analysis", Proceedings of the National Academy of Sciences, vol. 101, no. 7, 1 February 2004 (2004-02-01), pages 1933-1938, XP055064201, ISSN: 0027-8424, DOI: 10.1073/pnas.0305476101

### SEQUENCE LISTING

<110> Will, Stephen Newton, Nick
<120> SYMMETRIC PCR COUPLED WITH POST-PCR CHARACTERIZATION FOR THE IDENTIFICATION OF NUCLEIC ACIDS
<130> 21801 EP-RA
<150> US 60/695,991
   <151> 2005-06-30
<150> US 60/696,253
   <151> 2005-06-30
<150> US 60/696,293
   <151> 2005-06-30
<150> US 60/696,303
   <151> 2005-06-30
<160> 10
<170> Patent In version 3.3
<210> 1
   <211> 26
   <212> DNA
   <213> Artificial
<220>
   <223> HCV
<220>
   <221> misc_feature
   <222> (26)..(26)
   <223> E = t-butyl benzyl dA
<400> 1
<210> 2
   <211> 28
   <212> DNA
   <213> Artificial
<220>
   <223> HCV
<220>
   <221> misc feature
   <222> (28)..(28)
   <223> E = t-butyl benzyl dA
<400> 2
<210> 3
   <211> 29
   <212> DNA
   <213> Artificial
<220>
   <223> HCV
<220>
   <221> misc_feature
   <222> (1)..(1)
   <223> E = BHQ2
<220>
   <221> misc_feature
   <222> (2)..(3)
   <223> F = propynyl dU;
<220>
   <221> misc_feature
   <222> (8)..(9)
   <223> L = propynyl dC
<220>
   <221> misc_feature
   <222> (11)..(12)
   <223> F = propynyl dU;
<220>
   <221> misc_feature
   <222> (17)..(17)
   <223> F = propynyl dU;
<220>
   <221> misc_feature
   <222> (20)..(21)
   <223> F = propynyl dU;
<220>
   <221> misc_feature
   <222> (29)..(29)
   <223> J = cx-FAM;
<400> 3
<210> 4
   <211> 35
   <212> DNA
   <213> Artificial
<220>
   <223> HCV
<220>
   <221> misc_feature
   <222> (1)..(1)
   <223> E = CY5;
<220>
   <221> misc_feature
   <222> (11)..(11)
   <223> J = HEX
<220>
   <221> misc_feature
   <222> (35)..(35)
   <223> P = phosphate
<400> 4
<210> 5
   <211> 68
   <212> DNA
   <213> Artificial
<220>
   <223> HCV
<400> 5
   ccggaattgc caggacgacc gggtcctttc ttggattaac ccgctcaatg cctggagatt
   60
   tgggcgtg
   68
<210> 6
   <211> 68
   <212> DNA
   <213> Artificial
<220>
   <223> HCV
<400> 6
   ccggaattgc tgggaagact gggtcctttc ttggataaac ccactctatg cccagccatt
   60
   tgggcgtg
   68
<210> 7
   <211> 68
   <212> DNA
   <213> Artificial
<220>
   <223> HCV
<400> 7
   ccggaatcgc tggggtgacc gggtcctttc ttggagtaac ccgctcaata cccagaaatt 60
   tgggcgtg 68
<210> 8
   <211> 69
   <212> DNA
   <213> Artificial
<220>
   <223> HCV
<400> 8
   ccggaatcgc cgggatgacc gggtcctttc ttggattaaa cccgctcaat gcccggaaat 60
   ttgggtgtg 69
<210> 9
   <211> 68
   <212> DNA
   <213> Artificial
<220>
   <223> HCV
<400> 9
   ccggaattgc cgggatgacc gggtcctttc ttggataaac ccgctcaatg cccggagatt 60
   tgggcgtg 68
<210> 10
   <211> 68
   <212> DNA
   <213> Artificial
<220>
   <223> HCV
<400> 10
   ccggaattgc caggatgacc gggtcctttc ttggataaac ccgctcaatg cccggagatt 60
   tgggcgtg 68

## Description

### FIELD OF THE INVENTION

The current invention relates to the fields of nucleic acid chemistry and nucleic acid identification. More specifically, the invention relates to methods and compositions for amplifying and classifying specific nucleic acid sequences, which can be used for such purposes as diagnostics.

### BACKGROUND OF THE INVENTION

Numerous examples of the need for quick and reliable nucleic acid classification/ identification exist, especially in fields such as medicine. For example, many diseases and infections are caused by a number of, often related, pathogens. While the disease symptoms may present as similar, it can be of utmost importance to determine the actual causative pathogen in order to present an effective treatment. Not only is this true in terms of differentiation between different infectious species, but is also true, and can be even more difficult to resolve, when trying to discriminate between closely related agents, e.g., different strains of a pathogen such as the subtypes of hepatitis C virus (HCV).

Additionally, quick and reliable means of genotyping can be helpful in determining allele composition within and amongst individuals. For example, reliable classification of particular alleles in an individual can help in genetic counseling in humans and can even help in planning prophylactic treatment in instances when specific alleles are detected. Identification of particular alleles is also extremely useful in performing marker assisted selection, e.g., crop or animal breeding programs, identifying or genotyping pathogens and other organisms.

A number of different methods currently exist for detecting, identifying, genotyping, or quantifying various nucleic acids. Many of these rely on techniques that involve various binding actions between nucleic acid probes and the nucleic acid being examined such as restriction length fragment polymorphism analysis, sequencing, cleavage of probes that only occurs when specific target sequences are present, and the like.

However, there is a constant need for faster, simpler, and more flexible analysis tools. Ideal methods for classifying or genotyping of nucleic acid sequences would be easy to use and would involve the fewest manipulations of the components needed, thus, decreasing instances of error and reducing costs. The current invention provides these and other benefits which will be apparent upon examination of the current specification, claims, and figures.

### SUMMARY OF THE INVENTION

In various aspects herein, the invention comprises methods for classifying, identifying, quantifying and/or genotyping one or more nucleic acid targets in a sample (e.g., a blood sample or urine sample from a subject and/or a mixed sample of biological isolate(s) such as nucleic acid sample(s) in solution from a subject). Such methods comprise: performing asymmetric kinetic PCR in a reaction mixture containing one or more labeled 5'-nuclease probes and one or more labeled hybridization probes, wherein such 5'-nuclease probes can be the same in sequence as the hybridization probes or wherein the probes can be different in sequence from one another ; monitoring one or more growth curves from the kinetic PCR (kPCR), e.g., by tracking indicators such as fluorescence, to construct such growth curves from the kPCR for calculating a Cₜ (cycle threshold) value and thereby determining the copy number, the genotype or the target identity; modifying the temperature of the reaction mixture after the kPCR (e.g., over a range of temperatures, either increasing or decreasing and either over a continuous range or over a number of discrete temperatures) to cause a change in association between the one or more labeled hybridization probes and the one or more nucleic acid targets (e.g., melting or annealing); monitoring one or more fluorescent signals (or, in some embodiments, signals such as radiation, etc.) generated from the one or more labeled hybridization probes bound to the excess strand generated in the asymmetric kPCR, thereby producing a melting curve or annealing curve; and, correlating the melting curve or annealing curve, thus produced, to a melting or annealing curve of a completely complementary probe of one or more known nucleic acid targets, thus, identifying the one or more nucleic acid targets in the sample. Herein, the asymmetric kPCR comprises a first primer and at least a second primer, wherein the amount of the first primer is greater than the amount of the second primer and wherein the hybridization probe is present in greater amount than the second primer. Standard melting or annealing curves can optionally be determined from actual performance of the curves under set conditions or can be predicted based upon the nucleic acid compositions of the hybridization probes and the targets under set conditions without actual performance of the curve.

In some embodiments of such methods "identifying the one or more nucleic acid targets" can involve identifying organisms or organism strains having the target nucleic acid. For example, in various embodiments, identifying can entail identification of (or the presence of) particular bacteria or bacterial strains (e.g., staphylococci species, Mycobacteria species, Borrelia species, various enterococcus species, various *E. coli* strains, and the like), particular viruses or viral strains (e.g., HCV, HIV, influenza, HPV, HBV), particular fungi or fungal strains, or the presence of particular alleles or haplotypes (e.g., as used in genetic counseling to detect the presence and/or type of particular alleles).

In the various embodiments in the asymmetric kinetic PCR herein, the ratio of the first primer to the second primer can be selectively manipulated. Herein, the asymmetric kPCR comprises a first primer and at least a second primer, wherein the amount of the first primer is greater than the amount of the second primer (i.e., the limiting primer). Also, the one or more hybridization probe (which is complementary to the strand produced by the first, or non-limiting, primer in the asymmetric kPCR) exists in the reaction mixture in a greater amount than the second, or limiting, primer. For example, in some embodiments, the ratio (of first primer to second primer) can comprise at least 2:1, at least 3:1, at least 4:1, at least 5:1, at least 6:1, at least 7:1, at least 8:1, at least 9:1, at least 10:1, at least 15:1, at least 20:1, at least 50:1, at least 100:1, or at least 200:1 or more, depending upon, e.g., the desired end ratio of target nucleic acid strands (e.g., ratio of one strand to the other upon amplification).

In some embodiments, the methods comprise performing an asymmetric kPCR, followed by a thermal melting/annealing step, in the presence of an amplification indicator (e.g., an indicator of kinetic PCR amplification of the target sequences) that comprises a fluorescently labeled 5'- nuclease probe or otherwise labeled 5'-nuclease probe. Such probes maybe at least substantially complementary to, and hybridize with, the target nucleic acid sequences. In various embodiments, amplification is indicated by an increase in fluorescence, while in yet other embodiments, amplification is indicated by a decrease in fluorescence.

In certain embodiments, the same probe(s) are used as the 5'-nuclease probes and as the hybridization probes, thereby simplifying and streamlining the method steps. Thus, for example, the 5'- nuclease probes can be the same probes as the hybridization probes used to generate thermal melting/annealing curves as well. However, in yet other embodiments, the fluorescently labeled 5'-nuclease probes can be different probe(s) than the hybridization probes, e.g., in sequence, in labeling, etc.

In certain embodiments, the probes (e.g., the hybridization probes and/or the 5'-nuclease probes) can be completely complementary to a region of a nucleic acid target. In other embodiments, the probes can be partially complementary to a region of a nucleic acid target. Thus, for example, if a sample contained a number of different bacterial species all of which would have a target region amplified, but which target region comprised a different sequence in each species, a hybridization probe (whether or not it is the same as the 5'-nuclease probe) can be completely complementary to one of the species' regions and partially or not at all complementary to any of the other species' sequences; or the probe can be not completely complementary to any of the species' sequences while partially or not at all complementary to each of the other species' sequences, etc.

In certain embodiments of the invention, the change in association between the hybridization probe and the nucleic acid target can cause a change (e.g., increase) in fluorescence (which can then be optionally detected and quantified). In yet other embodiments, the change in association between a hybridization probe and a nucleic acid target can cause a decrease in fluorescence, which also can be detected and quantified.

Also disclosed is that the hybridization probes can optionally be present in the reaction mixture during amplification of the target nucleic acid (e.g., as when the hybridization probes are the same probes as the 5'-nuclease probes or as when the probes are different, but are both present in the reaction mixture prior to amplification), or the hybridization probes can be not present during the amplification of the target nucleic acid, e.g., as when the hybridization probes do not comprise the same probes as the 5'-nuclease probes and are added after kPCR.

The monitoring herein can occur over a range of temperatures, e.g., over a continuous range, or at discrete temperature points within a range.

In detecting and quantifying the kinetic PCR amplification of the target nucleic acids via the 5'-nuclease probes, a change in at least a first fluorescence can be monitored, while detecting and quantifying of the change in association of the hybridization probe with the target nucleic acid can be monitored by change in different fluorescence(s). Such different fluorescence(s) can optionally arise from different probes. In the embodiments wherein there are different probes (i.e., wherein the 5'-nuclease probe is different from the hybridization probe), each one can optionally be measured by a different fluorescence (e.g., from different fluorescent dyes) or other indicator. Other embodiments can involve measuring the same fluorescence for the kinetic PCR growth curve and for the change in association in the hybridization/melt curve since such curves produce the fluorescence at different times in the reaction sequence.

In some aspects, herein, the invention comprises methods wherein the one or more nucleic acid targets comprise a hepatitis C virus (HCV) nucleic acid (e.g., a nucleic acid from any HCV, such as HCV 1a, 1b, 2a, 2b, 3a, 3b, 4, 5, 6, or any other type, subtype and/or genotype). Thus, in such embodiments, identifying the HCV nucleic acid target identifies an HCV strain in the sample. Furthermore, in such embodiments, the hybridization probe(s) is substantially complementary with an HCV strain genotype (or with more than one HCV strain genotype). Such embodiments can comprise a single type of hybridization probe which shows different complementarity to different HCV strains, or multiple hybridization probes wherein a first probe is substantially complementary with a first HCV strain genotype and an at least second probe is substantially complementary with a second HCV strain genotype, etc. or multiple hybridization probes that are substantially complementary with multiple areas of those HCV strain genotypes. In certain embodiments the hybridization probe comprises a polynucleotide sequence of SEQ ID NO: 3

Also disclosed are kits for identifying one or more nucleic acid targets in a sample. Such kits can comprise: primers that are present in unequal amounts that are specific for amplification of one or more targets; one or more labeled hybridization probes (optionally fluorescently labeled) that are completely or partially complementary to at least one region of the nucleic acid target wherein the hybridization probes form hybridization complexes with the targets and which complexes have Tms; one or more labeled 5'-nuclease probes (optionally fluorescently labeled); and instructions for real-time asymmetric PCR amplification of the targets, for measuring the Tms of the hybridization complexes, and for identifying the nucleic acids based upon the Tms of the hybridization complexes. Also disclosed are kits, wherein the 5'-nuclease probe and the hybridization probe are the same probe. Also disclosed is, that the 5'-nuclease probe and the hybridization probe are not the same probe, e.g., they can differ in sequence, label, etc.

Also disclosed is a system, having one or more labeled hybridization probes (optionally fluorescently labeled); one or more labeled 5'-nuclease probes (optionally fluorescently labeled); two or more PCR primers that are present in unequal amounts and which are specific for amplification of one or more target nucleic acids; one or more container comprising the probes and primers (as well as kinetic PCR constituents such as buffers, salts and the like); one or more thermal modulator that is operably connected to the container and which can manipulate the temperature in the container; one or more detector that is configured to detect signals from the hybridization and/or 5'-nuclease probes (e.g., fluorescent signals); and, one or more controller that is operably connected to the detector and the thermal modulator and that can comprise one or more instruction sets for controlling the thermal modulator and the detector and that can also comprise one or more instruction sets for correlating the fluorescent signals and the temperature in the container with the presence of one or more target nucleic acid. Also disclosed is, that the 5'- nuclease probe in said kits is the same probe as the hybridization probe, while in some embodiments the 5'-nuclease probe and the hybridization probe are different from one another. Also disclosed is, that the system can further comprise a light source effective to excite the fluorescently labeled probe. Also disclosed is, that the system can further comprise one or more devices or subsystems for displaying or processing data obtained by the system.

Also disclosed is a reaction mixture comprising kinetic PCR primers present in unequal amounts specific for amplification of at least one nucleic acid target, one or more labeled 5'-nuclease probes and one or more labeled hybridization probes wherein the 5'-nuclease probes and the hybridization probes can be either the same probe or different probes. Herein, the primers are present in different amounts and the hybridization probes are present in a greater amount than the amount of the limiting primer (i.e., the primer present in the smaller amount).

Also disclosed are probes suitable for HCV genotyping, for example, a nucleic acid comprising a polynucleotide sequence of SEQ ID NO: 3.

These and other objects and features of the invention will become more fully apparent when the following detailed description is read in conjunction with the accompanying figures.

### DEFINITIONS

Unless defined otherwise, all technical and scientific terms used herein have the same meaning as commonly understood by one of ordinary skill in the art to which the invention pertains. The following definitions supplement those in the art and are directed to the current application and are not necessarily to be imputed to any related or unrelated case, e.g., to any commonly owned patent or application. Although any methods and materials similar or equivalent to those described herein can be used in the practice for testing of the present invention, the preferred materials and methods are described herein. Accordingly, the terminology used herein is for the purpose of describing particular embodiments only.

As used in this specification and the appended claims, the singular forms "a," "an," and "the" include plural referents unless the context clearly dictates otherwise. Thus, for example, reference to "an oligonucleotide" includes a plurality of oligonucleotides; reference to a "probe" includes mixtures of such probes, and the like.

As used herein, a **"sample"** refers to any substance containing or presumed to contain nucleic acid (e.g., from a bacteria, virus, etc.). The sample can be of natural or synthetic origin and can be obtained by any means known to those of skill in the art. Such sample can be an amount of tissue or fluid isolated from an individual or individuals, including, but not limited to, for example, skin, plasma, serum, whole blood, spinal fluid, saliva, peritoneal fluid, lymphatic fluid, aqueous or vitreous humor, synovial fluid, urine, tears, blood cells, blood products, semen, seminal fluid, vaginal fluids, pulmonary effusion, serosal fluid, organs, bronchio-alveolar lavage, tumors, paraffin embedded tissues, etc. Samples also can include constituents and components of *in vitro* cell cultures, including, but not limited to, conditioned medium resulting from the growth of cells in the cell culture medium, recombinant cells, cell components, etc. A nucleic acid can be obtained from a biological sample by procedures well known in the art.

The term **"nucleic acid"** refers to a polymer of monomers that can be corresponded to a ribose nucleic acid (RNA) or deoxyribose nucleic acid (DNA) polymer, or analog thereof. This includes polymers of nucleotides such as RNA and DNA, as well as modified forms thereof, peptide nucleic acids (PNAs), locked nucleic acids (LNA™s), and the like. In certain applications, the nucleic acid can be a polymer that includes multiple monomer types, e.g., both RNA and DNA subunits. A nucleic acid can be or include, e.g., a chromosome or chromosomal segment, a vector (e.g., an expression vector), an expression cassette, a naked DNA or RNA polymer, an amplicon, an oligonucleotide, a primer, a probe, etc. A nucleic acid can be e.g., single-stranded or double-stranded, or DNA:RNA hybrids, DNA and RNA chimeric structures. Unless otherwise indicated, a particular nucleic acid sequence optionally comprises or encodes complementary sequences, in addition to any sequence explicitly indicated. There is no intended distinction in length between the term "nucleic acid," "polynucleotide," and "oligonucleotide," and the terms can be used interchangeably herein unless the context clearly dictates otherwise. Such terms refer only to the primary structure of the molecule.

A nucleic acid is typically single-stranded or double-stranded and will generally contain phosphodiester bonds, although in some cases, as outlined herein, nucleic acid analogs are included that may have alternate backbones, including, for example, phosphoramide (Beaucage et al. (1993) Tetrahedron 49(10):1925 and the references therein; Letsinger (1970) J. Org. Chem. 35:3800; Sprinzl et al. (1977) Eur. J. Biochem. 81:579; Letsinger et al. (1986) Nucl. Acids Res. 14: 3487; Sawai et al. (1984) Chem. Lett. 805; Letsinger et al. (1988) J. Am. Chem. Soc. 110:4470; and Pauwels et al. (1986) Chemica Scripta 26:1419), phosphorothioate (Mag et al. (1991) Nucleic Acids Res. 19:1437 and U.S. Pat. No. 5,644,048), phosphorodithioate (Briu et al. (1989) J. Am. Chem. Soc. 111:2321), O-methylphophoroamidite linkages (Eckstein, Oligonucleotides and Analogues: A Practical Approach, Oxford University Press (1992)), and peptide nucleic acid backbones and linkages (Egholm (1992) L Am. Chem. Soc. 114:1895; Meier et al. (1992) Chem. Int. Ed. Engl. 31:1008; Nielsen (1993) Nature 365:566; and Carlsson et al. (1996) Nature 380:207). Other analog nucleic acids include those with positively charged backbones (Denpcy et al. (1995) Proc. Natl. Acad. Sci. USA 92:6097); non-ionic backbones (U.S. Pat. Nos. 5,386,023, 5,637,684, 5,602,240, 5,216,141 and 4,469,863; Angew (1991) Chem. Intl. Ed. English 30: 423; Letsinger et al. (1988) J. Am. Chem. Soc. 110:4470; Letsinger et al. (1994) Nucleoside & Nucleotide 13:1597; Chapters 2 and 3, ASC Symposium Series 580, "Carbohydrate Modifications in Antisense Research", Ed. Y. S. Sanghvi and P. Dan Cook; Mesmaeker et al. (1994) Bioorganic & Medicinal Chem. Lett. 4: 395; Jeffs et al. (1994) L Biomolecular NMR 34:17; Tetrahedron Lett. 37:743 (1996)) and non-ribose backbones, including those described in U.S. Pat. Nos. 5,235,033 and 5,034,506, and Chapters 6 and 7, ASC Symposium Series 580, Carbohydrate Modifications in Antisense Research, Ed. Y. S. Sanghvi and P. Dan Cook. Nucleic acids containing one or more carbocyclic sugars are also included within the definition of nucleic acids (Jenkins et al. (1995) Chem. Soc. Rev. pp169-176). Several nucleic acid analogs are also described in, e.g., Rawls, C & E News Jun. 2, 1997 page 35. These modifications of the ribose-phosphate backbone may be done to facilitate the addition of additional moieties such as labeling moieties, or to alter the stability and half-life of such molecules in physiological environments.

In addition to naturally occurring heterocyclic bases that are typically found in nucleic acids (e.g., adenine, guanine, thymine, cytosine, and uracil), nucleic acid analogs also include those having non-naturally occurring heterocyclic or other modified bases, many of which are described, or otherwise referred to, herein. In particular, many non-naturally occurring bases are described further in, e.g., Seela et al. (1991) Helv. Chim. Acta 74:1790, Grein et al. (1994) Bioorg. Med. Chem. Lett. 4:971-976, and Seela et al. (1999) Helv. Chim. Acta 82:1640. To further illustrate, certain bases used in nucleotides that act as melting temperature (Tm) modifiers are optionally included. For example, some of these include 7-deazapurines (e.g., 7-deazaguanine, 7-deazaadenine, etc.), pyrazolo[3,4-d]pyrimidines, propynyl-dN (e.g., propynyl-dU, propynyl-dC, etc.), and the like. *See,* e.g., U.S. Pat. No. 5,990,303, entitled "SYNTHESIS OF 7-DEAZA-2'-DEOXYGUANOSINE NUCLEOTIDES," which issued November 23,1999 to Seela. Other representative heterocyclic bases include, e.g., hypoxanthine, inosine, xanthine; 8-aza derivatives of 2-aminopurine, 2,6-diaminopurine, 2-amino-6-chloropurine, hypoxanthine, inosine and xanthine; 7-deaza-8-aza derivatives of adenine, guanine, 2-aminopurine, 2,6-diaminopurine, 2-amino-6-chloropurine, hypoxanthine, inosine and xanthine; 6-azacytosine; 5-fluorocytosine; 5-chlorocytosine; 5-iodocytosine; 5-bromocytosine; 5-methylcytosine; 5-propynylcytosine; 5-bromovinyluracil; 5-fluorouracil; 5-chlorouracil; 5-iodouracil; 5-bromouracil; 5-trifluoromethyluracil; 5-methoxymethyluracil; 5-ethynyluracil; 5-propynyluracil, 4-acetylcytosine, 5-(carboxyhydroxymethyl)uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 7-deazaadenine, 2-methyladenine, 2-methylguanine, 3-methylcytosine, 5-methylcytosine, N6-methyladenine, 7-methylguanine, 7-deazaguanine, 5-methylaminomethyluracil, 5-methoxyaminomethyl-2-thiouracil, beta-D mannosylqueosine, 5'-methoxycarboxymethyluracil, 5-methoxyuracil, 2-methylthio-N6- isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acidmethylester, 3-(3-amino-3-N-2-carboxypropyl) uracil, (acp3)w, 2,6- diaminopurine, and 5-propynyl pyrimidine, and the like.

Additional examples of modified bases and nucleotides are also described in, e.g., U.S. Pat. No. 5,484,908, entitled "OLIGONUCLEOTIDES CONTAINING 5-PROPYNYL PYRIMIDINES," issued January 16, 1996 to Froehler et al., U.S. Pat. No. 5,645,985, entitled "ENHANCED TRIPLE-HELIX AND DOUBLE-HELIX FORMATION WITH OLIGOMERS CONTAINING MODIFIED PYRIMIDINES," issued July 8, 1997 to Froehler et al., U.S. Pat. No. 5,830,653, entitled "METHODS OF USING OLIGOMERS CONTAINING MODIFIED PYRIMIDINES," issued November 3, 1998 to Froehler et al., U.S. Pat. No. 6,639,059, entitled "SYNTHESIS OF [2.2.1]BICYCLO NUCLEOSIDES," issued October 28, 2003 to Kochkine et al., U.S. Pat. No. 6,303,315, entitled "ONE STEP SAMPLE PREPARATION AND DETECTION OF NUCLEIC ACIDS IN COMPLEX BIOLOGICAL SAMPLES," issued October 16, 2001 to Skouv, and U.S. Pat. Application Pub. No. 2003/0092905, entitled "SYNTHESIS OF [2.2.1]BICYCLO NUCLEOSIDES," by Kochkine et al. that published May 15, 2003.

Such nucleic acid can be from a human or non-human mammal, or any other organism (e.g., plant, amphibian, bacteria, virus, mycoplasm, etc.), tissue, or cell line, or derived from any recombinant source, synthesized *in vitro* or by chemical synthesis. Again, the nucleic acid can be DNA, RNA, cDNA, DNA-RNA, locked nucleic acid (LNA), peptide nucleic acid (PNA), a hybrid or any mixture of the above. The nucleic acid can exist in a double-stranded, single-stranded or partially double-stranded form. The nucleic acids of the invention include both nucleic acids and fragments thereof, in purified or unpurified forms, including genes, chromosomes, plasmids, the genomes of biological material such as microorganisms, e.g., bacteria, yeasts, viruses, viroids, molds, fungi, plants, animals, humans, mycoplasms, and the like.

**"Corresponding"** as used herein, should be taken to mean identical to, or complementary to, a designated sequence of nucleotides in a nucleic acid. The precise meaning of the term will be evident to one of skill in the art from the context in which it is used.

A "complement" of a nucleic acid (or a nucleic acid that is "specific" in relation to another nucleic acid) refers to at least a nucleic acid segment that can combine in an antiparallel association or hybridize with at least a subsequence of that nucleic acid. The antiparallel association can be intramolecular, e.g., in the form of a hairpin loop within a nucleic acid, or intermolecular, such as when two or more single-stranded nucleic acids hybridize with one another. Certain bases not commonly found in natural nucleic acids may be included in the nucleic acids of the present invention and include, for example, inosine, 7-deazaguanine and those discussed above. Complementarity need not be perfect (i.e., nucleic acids can be "partially complementary"). Stable duplexes, for example, may contain mismatched base pairs or unmatched bases. Those skilled in the art of nucleic acid technology can determine duplex stability by empirically considering a number of variables including, for example, the length of a region of complementarity, base composition and sequence of nucleotides in a region of complementarity, ionic strength, and incidence of mismatched base pairs.

A **"subject"** refers to an organism. Typically, the organism is a mammalian organism, particularly a human organism. In certain embodiments, for example, a subject is a patient suspected of having a genetic disorder, disease state, or other condition.

Because mononucleotides can be arranged to create oligonucleotides in a manner such that the 5'-phosphate of one mononucleotide pentose ring is attached to the 3'-oxygen of its neighbor in one direction via a phosphodiester linkage, one end of an oligonucleotide is referred to as the "5'-end" if its 5'-phosphate is not linked to the 3'-oxygen of a mononucleotide pentose ring and one end is referred to as the "3'-end" if its 3'-oxygen is not linked to a 5'-phosphate of a subsequent mononucleotide pentose ring. As used herein, a nucleic acid sequence, even if internal to a larger oligonucleotide, also may be said to have 5' and 3'-ends.

A **"primer nucleic acid"** or **"primer"** is a nucleic acid that can hybridize to a target or template nucleic acid and permit chain extension or elongation using, e.g., a nucleotide incorporating biocatalyst, such as a polymerase under appropriate reaction conditions. Such conditions typically include the presence of one or more deoxyribonucleoside triphosphates and the nucleotide incorporating biocatalyst, in a suitable buffer ("buffer" includes substituents which are cofactors, or which affect pH, ionic strength, etc.), and at a suitable temperature. A primer nucleic acid is typically a natural or synthetic oligonucleotide (e.g., a single-stranded oligodeoxyribonucleotide, etc.). Although other primer nucleic acid lengths are optionally utilized, they typically comprise hybridizing regions that range from about 6 to about 100 nucleotides in length. Short primer nucleic acids generally utilize cooler temperatures to form sufficiently stable hybrid complexes with template nucleic acids. A primer nucleic acid that is at least partially complementary to a subsequence of a template nucleic acid is typically sufficient to hybridize with the template for extension to occur. The design of suitable primers for, e.g., the amplification of a given target sequence is well known in the art and described in the literature cited herein. A primer nucleic acid can be labeled, if desired, by incorporating a label detectable by, e.g., spectroscopic, photochemical, biochemical, immunochemical, chemical, or other techniques. To illustrate, useful labels include radioisotopes, fluorescent dyes, electron-dense reagents, enzymes (as commonly used in ELISAs), biotin, or haptens and proteins for which antisera or monoclonal antibodies are available. Many of these and other labels are described further herein and/or otherwise known in the art. One of skill in the art will recognize that, in certain embodiments, primer nucleic acids can also be used as probe nucleic acids. *See,* below.

As used herein, the term **"probe"** refers to an oligonucleotide (or other nucleic acid sequence) which can form a duplex structure with a region of a target nucleic acid (or amplicon derived from such target nucleic acid), due to partial or complete complementarity of at least one sequence in the probe with a sequence in the target nucleic acid under suitable conditions. The probe, in certain embodiments, does not contain a sequence complementary to sequence(s) of a primer in a 5'-nuclease reaction. As discussed herein, the probe can be labeled or unlabeled. The 3'-terminus of the probe optionally can be "blocked" to prohibit incorporation of the probe into a primer extension product. "Blocking" can be achieved by using non-complementary bases or by adding a chemical moiety such as biotin or a phosphate group to the 3'-hydroxyl of the last nucleotide, which can, depending upon the selected moiety, serve a dual purpose by also acting as a label for subsequent detection or capture of the nucleic acid attached to the label. Blocking can also be achieved by removing the 3'-OH or by using a nucleotide that lacks a 3'-OH such as a dideoxynucleotide, or by adding a bulky group that blocks extension by steric hindrance.

The term **"hybridizing region"** refers to that region of a nucleic acid that is exactly or substantially complementary to, and therefore hybridizes to, the target sequence. For use in a hybridization assay, e.g., for the discrimination of single nucleotide differences in sequence, the hybridizing region is typically from about 8 to about 100 nucleotides in length. Although the hybridizing region generally refers to the entire oligonucleotide, the probe may include additional nucleotide sequences that function, for example, as linker binding sites to provide a site for attaching the probe sequence to a solid support or the like. Probes herein can comprise "5'-nuclease" probes (typically comprising a fluorescent label(s) and typically a quencher as well) a FRET probe, or a molecular beacon, or the like, which can also be utilized to detect hybridization between the probe and target nucleic acids in a sample. In some embodiments, the hybridizing region of the probe is completely complementary to the target sequence. However, in general, complete complementarity is not necessary (i.e., nucleic acids can be partially complementary to one another); stable duplexes may contain mismatched bases or unmatched bases. Modification of the stringent conditions may be necessary to permit a stable hybridization duplex with one or more base pair mismatches or unmatched bases. Sambrook et al., Molecular Cloning: A Laboratory Manual, 3rd Ed., Cold Spring Harbor Laboratory Press, Cold Spring Harbor, N.Y. (2001), provides guidance for suitable modification. Stability of the target/probe duplex depends on a number of variables including length of the oligonucleotide, base composition and sequence of the oligonucleotide, temperature, and ionic conditions. One of skill in the art will recognize that, in general, the exact complement of a given probe is similarly useful as a probe. One of skill in the art will also recognize that, in certain embodiments, probe nucleic acids can also be used as primer nucleic acids.

A "5'-nuclease probe" refers to an oligonucleotide that comprises at least one light emitting labeling moiety and that is used in a 5'-nuclease reaction to effect target nucleic acid detection. In some embodiments, for example, a 5'-nuclease probe includes only a single light emitting moiety (e.g., a fluorescent dye, etc.). In certain embodiments, 5'-nuclease probes include regions of self-complementarity such that the probes are capable of forming hairpin structures under selected conditions. To further illustrate, in some embodiments a 5'-nuclease probe comprises at least two labeling moieties and emits radiation of increased intensity after one of the two labels is cleaved or otherwise separated from the oligonucleotide. In certain embodiments, a 5'-nuclease probe is labeled with two different fluorescent dyes, e.g., a 5'-terminus reporter dye and the 3'-terminus quencher dye or moiety. In some embodiments, 5'-nuclease probes are labeled at one or more positions other than, or in addition to, terminal positions. When the probe is intact, energy transfer typically occurs between the two fluorophores such that fluorescent emission from the reporter dye is quenched at least in part. During an extension step of a polymerase chain reaction, for example, a 5'-nuclease probe bound to a template nucleic acid is cleaved by the 5' to 3'-nuclease activity of, e.g., a *Taq* polymerase or another polymerase having this activity such that the fluorescent emission of the reporter dye is no longer quenched. Exemplary 5'-nuclease probes are also described in, e.g., U.S. Pat. No. 5,210,015, entitled "Homogeneous assay system using the nuclease activity of a nucleic acid polymerase," issued May 11, 1993 to Gelfand et al., U.S. Pat. No. 5,994,056, entitled "Homogeneous methods for nucleic acid amplification and detection," issued November 30,1999 to Higuchi, and U.S. Pat. No. 6,171,785, entitled "Methods and devices for homogeneous nucleic acid amplification and detector," issued January 9, 2001 to Higuchi. In other embodiments, a 5'-nuclease probe may be labeled with two or more different reporter dyes and a 3'-terminus quencher dye or moiety.

A **"hybridization probe"** herein refers to a labeled probe used in determination of Tm. In certain embodiments, the 5'-nuclease probe and the hybridization probe are the same. In other embodiments, the 5'-nuclease probe and the hybridization probe are separate probes and each can optionally comprise different labels and/or quenchers and each can optionally hybridize with different areas of the target nucleic acid. The hybridization probes herein are optionally dual labeled or quenched probes, wherein the quenchers may or may not be fluorescent. In the probes, energy transfer occurs between the reporter moiety and the quencher. Such probes can include, e.g., dark quenchers or the like. In some aspects, the hybridization probe is used as a genotyping probe, where the probe is used to assign a hybridization target to a particular genotype, *e.g.,* a viral genotype.

A **"label"** refers to a moiety attached (covalently or non-covalently), or capable of being attached, to a molecule, which moiety provides or is capable of providing information about the molecule (e.g., descriptive or identifying information about the molecule) or another molecule with which the labeled molecule interacts (e.g., hybridizes). Exemplary labels include fluorescent labels (including, e.g., quenchers or absorbers), non-fluorescent labels, colorimetric labels, chemiluminescent labels, bioluminescent labels, radioactive labels, mass-modifying groups, antibodies, antigens, biotin, haptens, enzymes (including, e.g., peroxidase, phosphatase), and the like. Labels may provide signals detectable by fluorescence, radioactivity, colorimetry, gravimetry, X-ray diffraction or absorption, magnetism, enzymatic activity, and the like. Labels can be used to provide a detectable (and optionally quantifiable) signal, and which can be attached to a nucleic acid or protein.

In certain embodiments of the invention, a label is a fluorescent dye or fluorophore. Typically, a particular fluorophore can emit light of a particular wavelength following absorbance of light of shorter wavelength. The wavelength of the light emitted by a particular fluorophore is characteristic of that fluorophore. Thus, a particular fluorophore can be detected by detecting light of an appropriate wavelength following excitation of the fluorophore with light of shorter wavelength. Fluorescent labels may include dyes that are negatively charged, such as dyes of the fluorescein family, or dyes that are neutral in charge, such as dyes of the carboxyrhodamine family, or dyes that are positively charged, such as dyes of the cyanine family or the rhodamine family. Other families of dyes that can be used in the invention include, e.g., polyhalofluorescein-family dyes, hexachlorofluorescein-family dyes, coumarin-family dyes, oxazine-family dyes, thiazine-family dyes, squaraine-family dyes, chelated lanthanide-family dyes, ALEXA FLUOR^{®} dyes, and BODIPY^{®}-family dyes. Dyes of the fluorescein family include, *e.g.,* FAM, HEX, TET, JOE, NAN and ZOE. Dyes of the carboxyrhodamine family include Texas Red, ROX, R110, R6G, and TAMRA. FAM, HEX, TET, JOE, NAN, ZOE, ROX, R110, R6G, and TAMRA are marketed by Perkin-Elmer (Foster City, CA), while Texas Red is marketed by Molecular Probes, Inc. (Eugene, OR). Dyes of the cyanine family include Cy2, Cy3, Cy3.5, Cy5, Cy5.5, and Cy7 and are marketed by Amersham GE Healthcare (Piscataway, NJ).

The term "quencher" as used herein refers to a chemical moiety that absorbs energy emitted from a fluorescent dye, or otherwise interferes with the ability of the fluorescent dye to emit light. In one embodiment, the quencher and fluorescent dye are both linked to a common polynucleotide. A quencher can re-emit the energy absorbed from a fluorescent dye in a signal characteristic for that quencher and thus a quencher can also be a "label." This phenomenon is generally known as fluorescent resonance energy transfer or FRET. Alternatively, a quencher can dissipate the energy absorbed from a fluorescent dye as heat (i.e., a non-fluorescent quencher). Molecules commonly used in FRET include, for example, fluorescein, FAM, JOE, rhodamine, R6G, TAMRA, ROX, DABCYL, and EDANS. Whether a fluorescent dye is a label or a quencher is defined by its excitation and emission spectra, and the fluorescent dye with which it is paired. For example, FAM is most efficiently excited by light with a wavelength of 494 nm, and emits light with a spectrum of 500 to 650 nm, and an emission maximum of 525 nm. FAM is a suitable donor label for use with, e.g., TAMRA as a quencher that has at its excitation maximum at 544 nm. Exemplary non-fluorescent quenchers that dissipate energy absorbed from a fluorescent dye include the Black Hole Quenchers™ marketed by Biosearch Technologies, Inc. (Novato, CA), Eclipse Dark Quenchers from Epoch Biosciences (Bothell, WA), and Iowa Black (Integrated DNA Technologies, Coralville, IA)

As defined herein, **"5' to 3**' **nuclease activity"** refers to that activity of a template-specific nucleic acid polymerase including either a 5' to 3'-exonuclease activity (traditionally associated with some DNA polymerases whereby nucleotides are removed from the 5' end of an oligonucleotide in a sequential manner, e.g., *E. coli* DNA polymerase I has this activity whereas the Klenow fragment does not), or a 5' to 3'-endonuclease activity (wherein cleavage occurs more than one phosphodiester bond (nucleotide) from the 5'-end), or both. Although not intending to be bound by any particular theory of operation, the preferred substrate for 5' to 3'-endonuclease activity-dependent cleavage on a probe-template hybridization complex is a displaced single-stranded nucleic acid, a fork-like structure. Hydrolysis typically occurs at the phosphodiester bond joining the displaced region with the base-paired portion of the strand, as discussed in Holland et al., 1991, Proc. Natl. Acad. Sci. USA 88:7276-80.

As used herein, the term **"thermostable"** and/or **"thermoactive" nucleic acid polymerase** refers to an enzyme that is relatively stable and/or active when heated as compared, for example, to nucleotide polymerases from *E. coli,* that catalyzes the polymerization of nucleoside triphosphates. Generally, the enzyme will initiate synthesis at the 3'-end of the primer annealed to a target sequence, and will continue synthesis of a new strand toward the 5'-end of the template, and if possessing a 5' to 3'-nuclease activity, will hydrolyze any intervening, annealed probe, thus, optionally releasing both labeled and unlabeled probe fragments. Such action will continue until synthesis terminates or probe fragments melt off the target sequence. A representative thermostable enzyme isolated from *Thermus aquaticus (Taq)* is described in U.S. Pat. No. 4,889,818 and a method for using it in conventional PCR is described in Saiki et al., 1988, Science 239:487-91. Those of skill in the art will be familiar with other similar enzymes. *Taq* DNA polymerase has a DNA synthesis-dependent, strand replacement 5'-3' exonuclease activity. *See* Gelfand, "Taq DNA Polymerase" in PCR Technology Principles and Applications for DNA Amplification, Erlich, Ed., Stockton Press, N.Y. (1989), Chapter 2. In solution typically there is little, if any, degradation of probes by such polymerases.

A "5'-nuclease reaction" or "5'-nuclease assay" of target or template, primer, and probe (e.g., 5'-nuclease probes, etc.) nucleic acids refers to the degradation of a probe hybridized to the template nucleic acid when the primer is extended by a nucleotide incorporating biocatalyst having 5' to 3'-nuclease activity. 5'-nuclease PCRs, also referred to as TaqMan reactions, are a type of PCR or RT-PCR that utilize a labeled oligonucleotide probe that binds to a single stranded nucleic acid target. In 5'-nuclease PCR, the probes are complementary (at least in part) to one or more regions of the target or targets. The probes can optionally be labeled with any of a number of moieties, but are typically labeled with a fluorescent label and a quencher. The arrangement of the moieties within the probe can also be varied in different embodiments. Because the fluorescent moiety is in close proximity to the quencher on the oligonucleotide probe, its fluorescence is inhibited.

In addition to the bound probe on the target nucleic acid, 5'-nuclease assays typically comprise amplification primers to allow amplification of the region of the target nucleic acid which comprises the bound probe. The enzyme (typically a DNA polymerase or the like) which has a 5'-exonuclease activity, cleaves the labeled probe when it extends the growing complementary polymer on the target nucleic acid. Once the probe is cleaved, the label on the probe is no longer in close proximity to its quencher and, thus, a discernable and quantifiable change in fluorescence can be observed. The observations can be made in real-time as the amplification proceeds.

Again, of course, many optional variations are possible within the basic 5'-nuclease scheme. For example, various permutations and applications of 5'-nuclease assays can be used to, e.g., determine copy number, genotype, allelic composition, etc. Further examples of such variations can be found in, e.g., U.S. Pat. No. 5,210,015 entitled "Homogeneous Assay System Using the Nuclease Activity of a Nucleic Acid Polymerase," to Gelfand, et al. issued May 11, 1993; U.S. Pat. No. 5,487,972 entitled "Nucleic Acid Detection by the 5'-3' Exonuclease Activity of Polymerases Acting on Adjacently Hybridized Oligonucleotides," to Gelfand et al., issued January 30, 1996; U.S. Pat. No. 5,804,375 entitled "Reaction Mixtures for Detection of Target Nucleic Acids," to Gelfand et al. issued September 8, 1998; and U.S. Pat. No. 6,214,979 entitled "Homogeneous Assay System," to Gelfand et al., issued April 10, 2001.

One measure of 5'-nuclease (e.g., TaqMan^{®}) assay data is typically expressed as the threshold cycle (Cₜ). Fluorescence levels are recorded during each PCR cycle and are proportional to the amount of product amplified to that point in the amplification reaction. The PCR cycle when the fluorescence signal is first recorded as statistically significant, or where the fluorescence signal is above some other arbitrary level *(e.g.,* the arbitrary fluorescence level, or AFL), is the threshold cycle (Cₜ).

In practice, a Cₜ value is the crossover point between the kinetic curve and an arbitrary threshold of fluorescence. The Cₜ value is inversely proportional to the logarithm of the initial number of template copies. Cₜ values are inversely proportional to the log of the initial nucleic acid template concentration and thus may be used to calculate target copy number.

A **"target nucleic acid"** refers to a nucleic acid that is amplified and/or identified by the current invention (e.g., an amplicon). Typically, a target nucleic acid, or "target," is one to which a probe and/or a primer(s) binds (e.g., a target optionally comprises one or more sequence of full complementarity with a primer and/or probe or comprises a sequence(s) with enough complementarity to one or more primer and/or probe to have such primer and/or probe bind to the target under appropriate environmental or reaction conditions). Typically, the identity, genotype, sequence, etc., of the target is to be identified by the methods of the present invention. A **"target primer"** and a **"target probe"** refer to a primer and probe, respectively, that can hybridize to the target nucleic acid.

A **"polymorphism"** refers to a site or sites of a nucleic acid that can comprise one of a plurality of genotypes. The polymorphism can be any polymorphism known to those of skill in the art including possible mutations, insertions or deletions. The polymorphism can be at one site within the nucleic acid or at multiple sites within the nucleic acid, and may encompass one nucleobase, such as a SNP, or more than one nucleobase. For the purposes of the present invention a **"polymorphism"** can refer to a polymorphism that is at one site of a nucleic acid or to one particular site of a multiple-site polymorphism. In certain embodiments, the polymorphism need not be well known or even known to those of skill in the art. The polymorphism can simply be any difference in nucleic acid sequence between a known (e.g., a control) nucleic acid and a target nucleic acid.

To determine **"percent complementarity"** or **"percent identity"** of two nucleic acid sequences, the sequences are aligned for optimal comparison purposes (e.g., gaps can be introduced in the sequence of a first nucleic acid sequence for optimal alignment with a second nucleic acid sequence). The nucleotides at corresponding nucleotide positions are then compared. When a position in the first sequence is occupied by a complementary nucleotide as the corresponding position in the second sequence, then the molecules are complementary at that position. Likewise, when a position in the first sequence is occupied by the same nucleotide as the corresponding position in the second sequence, then the molecules are identical at that position. The percent complementarity (or percent identity) between the two sequences is a function of the number of complementary positions (or identical positions) shared by the sequences divided by the total number of positions compared (i.e., percent complementarity = number of complementary overlapping positions/total number of positions of the shorter nucleotide x 100; and percent identity = number of identical overlapping positions/total number of positions of the shorter nucleotide x 100).

The determination of percent identity between two sequences can also be accomplished using a mathematical algorithm. A preferred example of a mathematical algorithm utilized for the comparison of two sequences is the algorithm of Karlin and Altschul, 1990, Proc. Natl. Acad. Sci. U.S.A. 87:2264-2268, modified as in Karlin and Altschul, 1993, Proc. Natl. Acad. Sci. U.S.A. 90:5873-5877. Such an algorithm is incorporated into the NBLAST program of Altschul et al., 1990, J. Mol. Biol. 215:403.

As used herein, the term **"Tₘ"** is used in reference to the "melting temperature." The melting temperature is the temperature at which one half of a population of double-stranded polynucleotides or nucleobase oligomers (*e.g*., hybridization complexes), in homoduplexes or heteroduplexes (ie., duplexes that are completely or partially complementary), become dissociated into single strands (under defined ionic strength, pH and nucleic acid concentration). The prediction of a Tₘ of a duplex polynucleotide takes into account the base sequence as well as other factors including structural and sequence characteristics and nature of the oligomeric linkages. Methods for predicting and experimentally determining Tₘ are known in the art.

For example, a Tₘ is traditionally determined by a melting curve, wherein a duplex nucleic acid molecule is heated in a controlled temperature program, and the state of association/dissociation of the two single strands in the duplex is monitored and plotted until reaching a temperature where the two strands are completely dissociated. The Tₘ is read from this melting curve. Alternatively, a Tₘ can be determined by an annealing curve, wherein a duplex nucleic acid molecule is heated to a temperature where the two strands are completely dissociated. The temperature is then lowered in a controlled temperature program, and the state of association/dissociation of the two single strands in the duplex is monitored and plotted until reaching a temperature where the two strands are completely annealed. The Tₘ is read from this annealing curve.

The terms **"stringent"** or **"stringent conditions,"** as used herein, denote hybridization conditions of low ionic strength and high temperature, as is well known in the art. *See, e.g.,* Sambrook et al., 2001, Molecular Closing: A Laboratory Manual, Third Sedition, Cold Spring Harbor Laboratory Press, Cold Spring Harbor, New York; Current Protocols in Molecular Biology (Ausubel et al., ed., J. Wiley & Sons Inc., New York, 1988); Tijssen, 1993, "Overview of principles of hybridization and the strategy of nucleic acid assays" in Laboratory techniques in biochemistry and molecular biology: Hybridization with nucleic acid probes (Elsevier). Generally, stringent conditions are selected to be about 5-30 °C lower than the thermal melting point (Tₘ) for the specified sequence at a defined ionic strength and pH. Alternatively, stringent conditions are selected to be about 5-15 °C lower than the Tₘ for the specified sequence at a defined ionic strength and pH. For example, stringent hybridization conditions will be those in which the salt concentration is less than about 1.0 M sodium (or other salts) ion, typically about 0.01 to about 1 M sodium ion concentration at about pH 7.0 to about pH 8.3 and the temperature is at least about 25°C for short probes (e.g., 10 to 50 nucleotides) and at least about 55°C for long probes (e.g., greater than 50 nucleotides). Stringent conditions may also be modified with the addition of hybridization destabilizing agents such as formamide. An exemplary non-stringent or low stringency condition for a long probe (e.g., greater than 50 nucleotides) would comprise a buffer of 20 mM Tris, pH 8.5, 50 mM KCl, and 2 mM MgCl₂, and a reaction temperature of 25°C.

As used herein, the expression **"hepatitis C virus type"** refers to the categorization of a hepatitis C virus (HCV) based on its genomic organization. The categorization of an HCV isolate into a particular type category reflects its genomic relatedness to other HCV isolates and its relatively lesser relatedness to other HCV isolates. As used herein, HCV typing nomenclature is consistent with the widely adopted nomenclature proposed by Simmonds et al (1994) Letter, Hepatology 19:1321-1324. See, also, Zein (2000) "Clinical Significance of Hepatitis C Virus Genotypes," Clinical Microbiol. Reviews 13(2):223-235; Maertens and Stuyver (1997) "Genotypes and Genetic Variation of Hepatitis C Virus," p. 182-233, In Harrison, and Zuckerman (eds.), The Molecular Medicine of Viral Hepatitis, John Wiley & Sons, Ltd., Chichester, England.). The system of Simmonds *et al* (1994) places the known HCV isolates into one of eleven (11) HCV genotypes, namely genotypes 1 through 11. Each genotype is further subdivided into groupings termed subtypes that reflect relatedness among strains of the same genotype. An HCV subtype is written by a lowercase roman letter following the genotype, *e.g*., subtype 1a, subtype 1c, subtype 6a, etc. Genetic variants found within an individual isolate are termed quasispecies. Approximately 78 HCV subtypes encompassing all 11 genotypes are known worldwide; the number of subtypes is not static; as more HCV isolates are studied and sequenced, it is likely that additional subtypes (and possibly genotypes) may be recognized.

As used herein, the term "HCV virus type" can refer to either HCV genotypes or HCV subtypes. As used herein, the term "HCV typing" means assigning the experimental (e.g., unknown type) HCV to a known genotype (*e*.*g*., 1, 2, 3, 4, 5 or 6, or a subset thereof) or assigning the experimental HCV to a known subtype (*e.g*., 1a, 1b, 1c, 2a, 2b, 2c, etc., or a subset thereof).

Some reports (see, *e.g.,* Robertson et al., (1998) Arch. Virol., 143(12):2493-2503) suggest that viral genomic organization is best represented by the creation of viral clades, reflecting the observation that some HCV genotypes are more closely related to each other than to other HCV genotypes. In this system, clades 1, 2, 4 and 5 correspond to genotypes 1, 2, 4 and 5, while clade 3 comprises genotypes 3 and 10, and clade 6 comprises genotypes 6, 7, 8, 9 and 11. The description of the present invention does not use the clade nomenclature.

As used herein, the term **"kit"** is used in reference to a combination of articles that facilitate a process, method, assay, analysis or manipulation of a sample. Kits can contain written instructions describing how to use the kit (e.g., instructions describing methods for HCV genotyping), chemical reagents or enzymes required for the method, primers and probes, as well as any other components. In some embodiments, the present invention provides kits for HCV typing employing RT-PCR. These kits can include, for example, reagents for sample collection *(e.g.,* the collection of a blood sample), reagents for the collection and purification of RNA from blood, a reverse transcriptase, primers suitable for reverse transcription and first strand and second strand cDNA synthesis to produce an HCV amplicon, a thermostable DNA-dependent DNA polymerase and free deoxyribonucleotide triphosphates. In some embodiments, the enzyme comprising reverse transcriptase activity and thermostable DNA-dependent DNA polymerase activity are the same enzyme, *e.g., Thermus* sp. ZO5 polymerase or *Thermus thermophilus* polymerase.

The practice of the present invention employs, unless otherwise indicated, conventional techniques of molecular biology, microbiology and recombinant DNA techniques, which are within the skill of the art. Such techniques are explained fully in the literature. *See, e.g.,* Sambrook et al., 2001, Molecular Cloning: A Laboratory Manual, Third Edition, Cold Spring Harbor Laboratory Press, Cold Spring Harbor, New York; Oligonucleotide Synthesis (M. J. Gait, ed., 1984); Nucleic Acid Hybridization (B. D. Hames & S. J. Higgins, eds., 1984); A Practical Guide to Molecular Cloning (B. Perbal, 1984); and a series, Methods in Enzymology (Academic Press, Inc.), etc. Those of skill in the art will be familiar with myriad similar references.

### BRIEF DESCRIPTION OF THE DRAWINGS

**Figure 1** provides a diagram illustrating the placement of primers and probe upon nucleic acid strands during asymmetric PCR.
**Figure 2** provides a stained agarose gel comparing nucleic acid amplification in asymmetric PCR versus symmetric PCR, using different primer ratios with 2x10⁵ copies of cytomegalovirus (CMV) DNA as the target nucleic acid.
**Figure 3** provides growth curves generated during an asymmetric kPCR
**Figure 4** provides the first derivative of melting curves using different primer ratios in an asymmetric kPCR.
**Figure 5** provides an alignment of a variable region from six different HCV genotypes and a corresponding exemplary hybridization probe.
**Figure 6** provides growth curve data generated in asymmetric kPCRs of six different HCV genotypes, using a HEX-labeled probe directed to a conserved region of the HCV genome.
**Figure 7** provides growth curve data generated in asymmetric kPCRs of six different HCV genotypes, using a FAM-labeled probe directed to a variable region of the HCV genome.
**Figure 8** provides second derivative plot of annealing curves generated in asymmetric kPCRs of six different HCV genotypes using a probe directed to a variable region of the HCV genome.

### DETAILED DESCRIPTION

The current invention comprises methods, compositions, kits, systems, and reaction mixtures useful for detection, classification, and quantification of particular nucleic acid sequences within samples (e.g., within clinical samples such as blood or sputum or within isolated DNA or RNA samples, etc.). The current invention can be used for, e.g., classifying, identifying, quantifying, and/or genotyping nucleic acids from various strains of bacteria, viruses, fungi, etc., especially those involved in infections and diseases (e.g., of humans, livestock, plants, etc.).

The methods of the invention utilize real-time, or kinetic, polymerase chain reaction amplification of target nucleic acid sequences performed in an asymmetric manner in the presence of a labeled oligonucleotide probe (a 5'-nuclease probe). The asymmetric kinetic PCR results in a mixture of single-stranded and double-stranded amplification products having an excess of one strand over the other. When the kinetic PCR is completed, melting/annealing curves are created using one or more hybridization probes. These melting/annealing curves are used to generate a Tm for the hybridization probe, which may be used to identify the target nucleic acid in the sample. In some embodiments, the hybridization probe is present in the kinetic PCR; in other embodiments, the hybridization probe is added after completion of the amplification reaction. In certain embodiments the 5'-nuclease probes and the hybridization probes (e.g., probes which are used in construction of thermal curves), comprise different probes (e.g., different in sequence and/or in binding site, etc.) while in certain other embodiments, the 5'-nuclease probes can also act as hybridization probes.

As a result of embodiments wherein the asymmetric kinetic PCR comprises an amount of the 5'-nuclease probe greater than the amount of the limiting PCR primer, a portion of a 5'-nuclease probe that hybridizes to the excess strand is left uncleaved at the finish of the amplification reaction. This "left-over" probe can then be used to create annealing or melt curves to determine Tms, which can aid in the determination of identity of the target nucleic acid that was amplified.

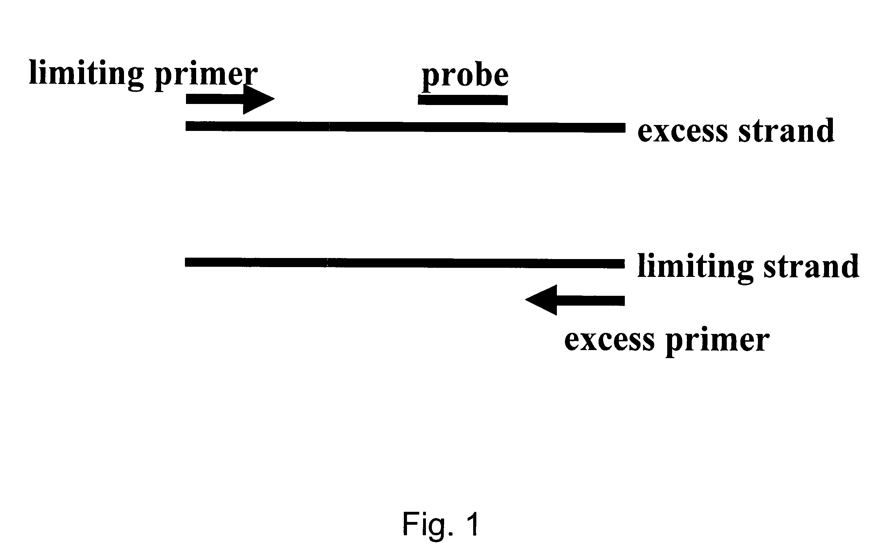

Incorporating a melting or annealing step into an asymmetric kinetic PCR requires several considerations. First, in a conventional kinetic PCR the probe target typically is a double-stranded amplicon (as opposed to just a single-stranded complement), thus, there are significant competitive effects that arise from strand reannealing, especially when the amplicon concentration rises in later PCR cycles. Second, since in any kinetic PCR the probe is degraded by the 5' to 3'-nuclease activity of the DNA polymerase, a supply of such probe must be ensured at the end of PCR in order to generate the melting/annealing curve. Both of these challenges can be met in the invention by performing asymmetric kinetic PCR. In asymmetric kinetic PCR the concentration of the primer used to generate the strand to which the 5'-nuclease probe (e.g., the probe used in the 5'-nuclease reaction) binds is in excess over the concentration of the other primer. This, thus, produces a greater amount of one strand of the amplicon (or amplified target nucleic acid) than the other strand. This, in turn, allows the 5'-nuclease probe to bind to its target (on the strand that is in excess) without the other amplicon strand competing it off. The second, or limiting, primer also limits the amount of probe cleaved during PCR, thus ensuring that there is probe left behind to perform the post-PCR melting step (in embodiments wherein additional hybridization probe is not added post-PCR and/or wherein a different hybridization probe is used). *See* **Figure 1****.** It is surprising how little growth curve (i.e., cleaved) signal is lost during the PCR under asymmetric conditions. For only a modest drop in growth curve signal, enough probe can be left behind to give the additional melting data.

5'-Nuclease probes have typically been used to generate fluorescent signal during real-time or kinetic PCR in the form of a growth curve. From such growth curves a Cₜ (threshold cycle) value is calculated and used, in either a quantitative or qualitative algorithm, to give a desired result such as a copy number, genotype, or target identity. During this process, the 5'-nuclease probe is cleaved by an enzyme with 5'-nuclease activity, thus, generating a variety of DNA fragments, some of which will be labeled with the fluorescent reporter. Once these fragments are generated they can no longer participate in further signal generation. However, by using asymmetric kinetic PCR, full-length intact 5'-nuclease probe can be left behind after the PCR is complete, e.g., when asymmetric PCR is performed, and/or when an excess of probe over limiting primer is added. Again, by arranging for sufficient probe to be left after the growth curve is generated, further information can be provided about the target that has been amplified by performing a melting step or an annealing step. A particular Tm could indicate that the probe and the target match perfectly and therefore the target nucleic acid belongs to a certain virus group or is a certain virus, virus type, or virus subtype, etc. Alternatively, the Tm could be lower than that which would result from a perfectly matched probe and target. The degree of decrease in the Tm can indicate (or can allow to be calculated) what mismatches are present, and thus, that the target sequence belongs to a different virus group.

Tm's generated from the current invention can therefore provide additional information to that obtained from the growth curve data. For example, viral identity in a positive sample in a blood screening assay; bacterial or fungal identity in a microbiology assay; or genotyping, e.g., in an HCV positive sample can all be performed with various embodiments of the current invention. In certain embodiments, the steps of the invention are achieved in a single-tube, homogeneous assay, without removing the cap. This greatly adds to the value of both existing and future assays requiring only minor changes in primer and probe concentrations and using existing commercially available 5'-nuclease reaction platforms.

During the course of typical 5'-nuclease assays, the probe is cleaved by an enzyme having 5'-nuclease activity (commonly a DNA polymerase such as *Taq* polymerase, etc.). Cleavage of the probe creates a fluorescent signal during real-time or kinetic PCR. Such signals are used to create a growth curve (or amplification curve) that can be used to calculate a C_{T} and to determine, e.g., presence or absence, copy number, genotype, target identity, etc. of the nucleic acid from a quantitative or qualitative algorithm. After the probe is cleaved, it typically can no longer participate in further signal generation. The current invention provides a 5'-nuclease assay in which a certain amount of probe is left over after the 5'-nuclease assay is performed, such that the remaining probe can be used to generate thermal melting (or thermal annealing) curves because of the change in fluorescence when the labeled probe hybridizes or melts from the target nucleic acid. Information about the target nucleic acid (e.g., sequence, and thus, genotype, identity, etc.) can be determined from the Tm's generated, separate and apart from the information generated by the growth curve. To ensure that sufficient probe remains, asymmetric PCR is performed with an excess of one primer in comparison to the other.

### ASYMMETRIC PCR

The current invention utilizes asymmetric PCR to ensure that sufficient probe remains after kinetic PCR amplification and that ssDNA is present for the probe to bind (either 5'-nuclease probe or a non-5'-nuclease hybridization probe). Figure 1 schematically illustrates asymmetric PCR as utilized herein. As can be seen in Figure 1, the probe binds to the excess target nucleic acid strand, i.e., the strand that is generated by the excess primer. PCR occurs after the primers bind, and double stranded target is synthesized until the limiting primer is depleted. After the limiting primer is depleted, linear amplification continues to generate the excess single strand. Such excess strand (i.e., the strand that is in excess in relation to the other) is the strand to which the probe binds. The strand having the bound probe, i.e., the excess strand, is the one involved in generating signal from 5'-nuclease release of label during the kinetic PCR amplification and is the one involved in generation of signal involving hybridization in the post-PCR melt/anneal steps.

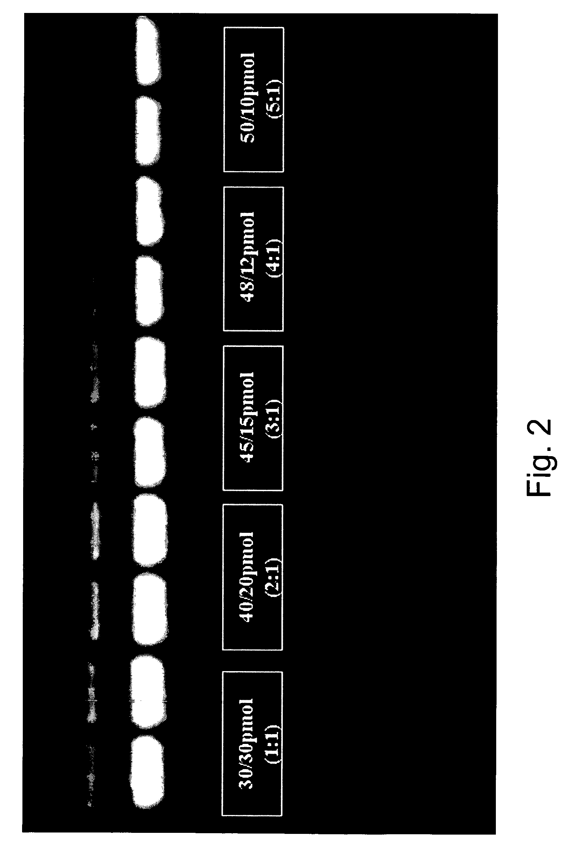

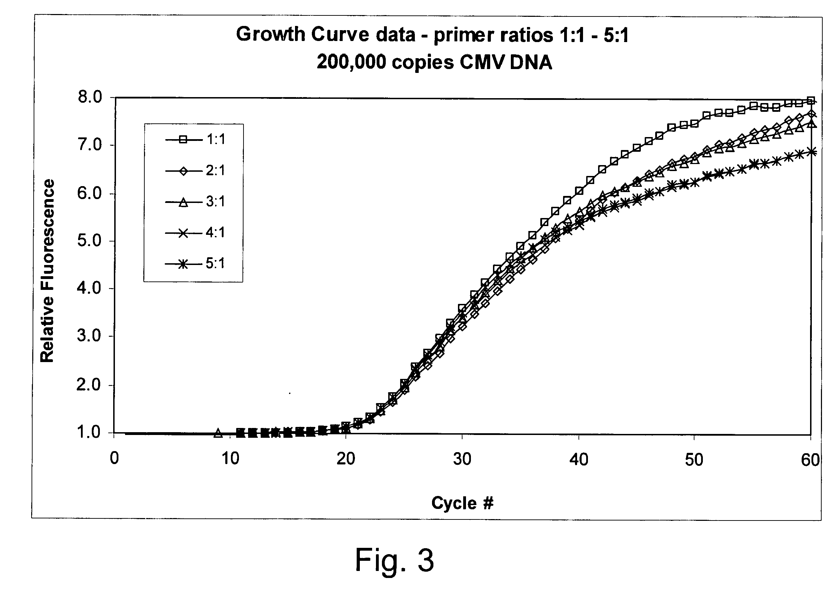

A factor in the use of asymmetric PCR concerns the effects of low concentration of the limiting primer on generation of the target nucleic acid and on generation of the signal during PCR. To ensure that asymmetric PCR does not unduly influence the current invention, different primer ratios from 1:1 to 5:1 were evaluated in a CMV 5'-nuclease assay. **Figure 2** shows a photograph of a stained gel which shows that the total amount of target nucleic acid produced was reduced when the PCR went from symmetric PCR (here with 30 pmol of each primer) to asymmetric PCR (here 50 pmol of one primer and 10 pmol of the other primer). However, the growth curves produced from the asymmetric PCR, shown in **Figure 3****,** showed very minimal effect on fluorescent signal generation from the varying primer amounts. This result demonstrates that probe cleavage in asymmetric PCR is sufficient to generate a useful growth curve. In certain embodiments, different primer ratios are optionally used. For example, some embodiments can have primer ratios ranging from at least 2:1, at least 3:1, at least 4:1, at least 5:1, at least 6:1, at least 7:1, at least 8:1, at least 9:1, at least 10:1, at least 15:1, at least 20:1, at least 50:1, at least 100:1, or at least 200:1 or more.

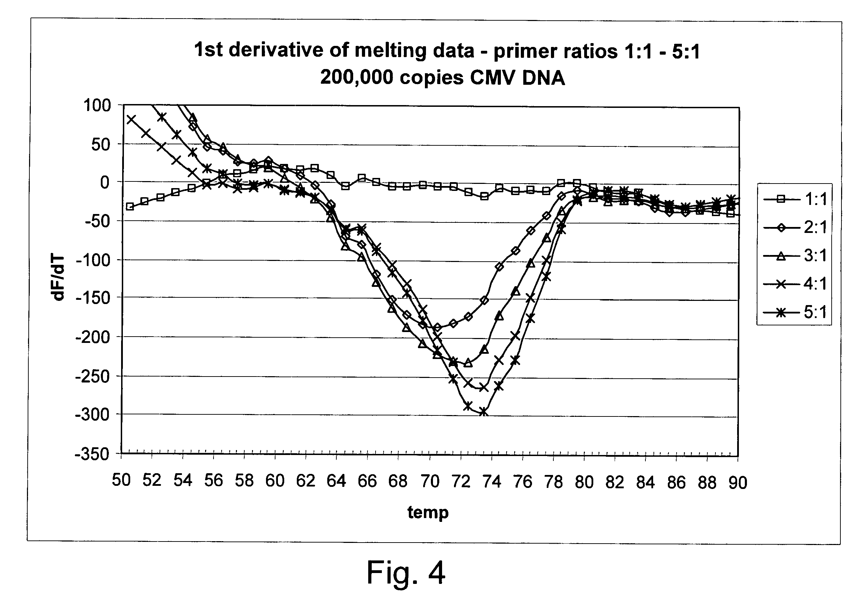

**Figure 4** shows post-PCR melting curves, e.g., as would be done to determine identity or genotype of a target, etc. The effect of the different primer ratios on signal generation can be seen from the figure. Fully symmetric PCR (i.e., wherein the primers are present in equal amounts) gives no melting signal at all, most likely because the majority of the probe has been cleaved during the amplification process or because of competition for amplicon strand re-annealing. Asymmetric PCR below a ratio of 2:1 also gives no signal, likely for similar reasons. With primer ratios from 2:1 to 5:1, differing amounts of melt signal are observed. The magnitude of signal generated with the asymmetric primers is approximately proportional to the primer ratio. It will be appreciated that in certain embodiments, the amount of probe added is greater than the amount of the limiting primer in the asymmetric PCR. Furthermore, it will be noted that the Tm of the probe also shifts depending on primer ratio. This is most likely due to the different probe concentrations left after PCR since Tm is dependent on oligonucleotide concentration.

Different probe concentrations can be seen by running reactions on laser induced fluorescent capillary electrophoresis, which detects FAM fluorescence. As the primer ratio increases, more full length uncleaved probe is left behind after the PCR is complete.

### TARGET NUCLEIC ACID

As explained above, the target nucleic acid(s) herein can be any nucleic acid of any length whose genotype, identity, sequence, or the like is to be determined. In certain embodiments, the general identity of the target nucleic acid is known but for the genotype of one or more polymorphisms. For example, a sample may be known to be HCV, but the exact viral type or subtype is unknown. In yet other embodiments, even the general identity of the target may not be confidently known. The methods of the present invention can be used to identify the genotype of one or more of the polymorphisms. The polymorphisms or particular sequences to be determined can be of any size and at any location in a target nucleic acid that is between the primer binding sites. Typically, the polymorphisms are not at the ends of the target nucleic acid, but even those at the ends can be genotyped or determined with the methods of the current invention.

The target nucleic acid can be obtained from any source known to those of skill in the art. For example, it can be obtained from a biological sample, e.g., those described above or others. It can be a nucleic acid from any natural source, e.g., including a human or a human pathogen or any other natural source. Also, in certain embodiments the target nucleic acid can be produced by synthetic, semi-synthetic or recombinant techniques.

In certain embodiments of the invention, the target nucleic acid can be amplified by a number of methods known to those of skill in the art. Such methods are described, for example in Saiki et al., 1988, Science 239:487-91. In certain embodiments, amplification techniques can be advantageously employed to introduce or alter nucleotide sequences in a target nucleic acid. For instance, if a polymorphism to be identified or genotyped is at or near an end of the target nucleic acid, additional nucleotide sequences can be added to the end of the target nucleic acid to facilitate the methods of the instant invention.

### OLIGONUCLEOTIDE PROBES

The current invention involves the selection and use of labeled probes to hybridize to specific regions of target nucleic acid. The choice of such probes, e.g., not only their specific sequence, but to which target area they should be targeted, depends to a large degree upon the specific target being assayed. Currently, numerous software programs and other protocols exist to help in choosing and designing 5'-nuclease probes or other hybridization probes for various applications. Such programs and protocols can optionally be utilized with the current invention to choose and design probes for the asymmetric kinetic PCR/melting curve assays herein. Of course, visual design and placement of probes is also quite applicable to the current invention as well. The parameters for design of not only 5'-nuclease probes, but various hybridization probes to construct thermal melting/annealing curves are extremely familiar to those of skill in the art. Programs which utilize different algorithms and parameter sets and which are useful for such design include, e.g., Visual OMP (DNA Software, Inc., Ann Arbor, MI), Oligo 6 (Stratagene, La Jolla, CA), Sequencher (Gene Codes, Ann Arbor, MI), and DNAStar (DNAStar, Inc., Madison, WI).

In certain embodiments of the invention, the 5'-nuclease probe and/or the hybridization probe is labeled with a label that facilitates the determination of the identity, genotype, or sequence of a target. The probe nucleotide sequence can be of any length sufficient to appropriately hybridize to target region(s) on the target nucleic acid and that can be used to genotype or identify the target nucleic acid. The length of the target probe typically will be chosen to give sufficient thermodynamic stability to ensure hybridization of the probe to its target at the temperature of the annealing step of PCR, etc. For example, probes with non-conventional DNA bases may optionally be longer or shorter than those with conventional DNA bases. As another example, probes with A/T-rich sequences will be longer than those with G/C-rich sequences, where the Tms are identical. The site of the polymorphism or target region can be at any location within the probe nucleotide sequence. In some embodiments, the site of such regions is not at the 5'-end of the probe nucleotide sequence.

No matter the individual sequence design of the probes used herein, a number of different approaches exist. For example, the same probe may be used for both the 5'-nuclease growth curve and for the melting/annealing curve. Such a probe is optionally targeted to one region of target nucleic acid that is common (at least in some variation) to all possible samples to be assayed. For example, in genotyping a virus subtype (e.g., such as illustrated in the Example herein) from amongst a number of different possibilities, consideration will typically be taken in choosing polymorphic areas of the target nucleic acid that contain sequence motifs unique to each subtype. Thus, the probe will differentially hybridize to such target region depending on the genotype of the target nucleic acid. Again, such varying degree of match between the probe and the target in a sample and/or amongst the different genotypes in a sample, influences the Tm curves generated in the assay and so can allow identification of the nucleic acid sample(s).

Thus, in a hypothetical example, a sample may contain a mixture of any number of unidentified nucleic acid types. For example, the sample may contain possibly related viral strains (e.g., HCV types/subtypes). Probes (5'-nuclease and/or 5'-nuclease and other separate hybridization probes) can be designed for a particular polymorphic region on the HCV nucleic acid. Mismatches between the probe(s) and the various polymorphic target regions would lead to different Tms (under similar conditions) between the probe(s) and the different target nucleic acids. Based on the sequences of the suspected targets, the expected Tms (under defined conditions) could be calculated.

The actual Tm curves generated can then be compared against predicted curves or previously generated standard curves. For example, if virus 1 were expected to produce a Tm of X under defined conditions and sample 1 did not produce a Tm of X under those conditions, then virus 1 could be ruled out as a possible component of the sample mixture. Correspondingly, if the Tm produced from an unknown in a sample produced a Tm of Y, the sequence of the hybridization target area could be calculated based on the known probe sequence, the assay conditions, etc. Determination of the unknown in the sample (e.g., through comparison of calculated sequences against sequence databases of known virus, etc.) could then be done.

In certain embodiments herein, the invention can comprise probe(s) used for the 5'-nuclease growth curve portion of the analysis that are different from the probe(s) used for the Tm curve generation portion of the analysis. Such an arrangement could be useful in situations wherein, e.g., the region of polymorphism on the target nucleic acid is so diverse that construction of a stable 5'-nuclease probe which would work within amplification conditions is not feasible. Thus, for example, a plurality of completely complementary or substantially complementary probes or partially complementary probes, each specific for at least one of the target nucleic acids, could be used for the 5'-nuclease analysis (thus ensuring the measurement of amplification of all desired targets) while a more generalized probe sequence (e.g., one that would hybridize to a number of related sequences) could be used for generation of Tm curves from a number of different target sequences. Also, it will be appreciated that while certain embodiments of the invention contain 5'-nuclease probes (e.g., in construction of the kinetic PCR growth curve), in those embodiments wherein different probes are used for the growth curve and the melt curve, the probes used in construction of the melt curve need not be 5'-nuclease probes (i.e., such probes are not necessarily hydrolyzed in certain embodiments). Whether the probes are hydrolyzed or not, can influence their design. For example, some 5'-nuclease probes may be about 30 bp in length and contain a dye internal to the probe ends. However, in probes that are not hydrolyzed the length may be shorter, the dye may be located on a terminus, etc.

In certain embodiments of the invention, the 5'-nuclease probe and/or the hybridization probe is labeled with a label that facilitates the determination of the identity, genotype, or sequence of an oligonucleotide fragment. The probe nucleotide sequence can be of any length sufficient to appropriately hybridize to target region(s) on the target nucleic acid and that can be used to genotype or identify the target nucleic acid. The length of the target probe typically will be chosen to give sufficient thermodynamic stability to ensure hybridization of the probe to its target at the temperature of the annealing step of PCR, etc. For example, probes with non-conventional DNA bases may optionally be longer or shorter than those with conventional DNA bases. As another example, probes with A/T-rich sequences will be longer than those with G/C-rich sequences. For example, since the current invention utilizes 5'-nuclease reactions, which will cleave the 5'-nuclease probes, longer probes can be utilized than in non-5'-nuclease reactions. Such longer probes can allow finer discrimination between sequences in Tm analysis and a bigger diagnostic window. The site of the polymorphism or target region can be at any location within the probe nucleotide sequence. In some embodiments, the site of such regions is not at the 5'-end of the probe nucleotide sequence.

Furthermore, in embodiments wherein different probes are used for the 5'-nuclease and Tm curves, the probes are optionally added at the same time, typically at the start of the analysis. Other embodiments herein can optionally comprise addition of multiple probes at different times within the analysis. For example, 5'-nuclease curve probe(s) can be added before the hybridization probe(s) are added.

In some embodiments, the probe nucleotide sequence (whether 5'-nuclease or hybridization) is identical or complementary to the target region. However, in many embodiments, the probe nucleotide sequence can have less than 100% identity or complementarity to the target nucleotide region. In certain embodiments of the invention, the probe nucleotide sequence can have 99%, 98%, 97%, 96%, 95%, 90%, 85% or 80% or less complementarity or identity to the target nucleotide region. In certain embodiments of the invention, the probe nucleotide sequence hybridizes to the target nucleotide region under stringent or highly stringent conditions. In other embodiments of the invention, the probe nucleotide sequence hybridizes to the target nucleotide region under low stringency conditions.

In certain embodiments of the invention, the probe can comprise one or more label, and optionally one or more quencher. In convenient embodiments, the label can be a label that facilitates the determination of the kinetic growth curve and/or the Tm curve.

The probe can be labeled by incorporating moieties detectable by spectroscopic, photochemical, biochemical, immunochemical, or chemical means. The method of linking or conjugating the label to the oligonucleotide probe depends, of course, on the type of label(s) and/or quencher(s) used and the position of the such on the probe. Typically, labels provide signals that are detectable by fluorescence, but some embodiments can also comprise labels detectable by, e.g., radioactivity, colorimetry, gravimetry, X-ray diffraction or absorption, magnetism, enzymatic activity, and the like. *See* above.

Fluorescent labels can include dyes that are negatively charged, such as dyes of the fluorescein family, or dyes that are neutral in charge, such as dyes of the rhodamine family, or dyes that are positively charged, such as dyes of the cyanine family. Dyes of the fluorescein family include, e.g., FAM, HEX, TET, JOE, NAN and ZOE. Dyes of the rhodamine family include Texas Red, ROX, R110, R6G, and TAMRA. FAM, HEX, TET, JOE, NAN, ZOE, ROX, R110, R6G, and TAMRA are marketed by Perkin-Elmer (Foster City, CA), and Texas Red is marketed by Molecular Probes, Inc. (Eugene, OR). Dyes of the cyanine family include Cy2, Cy3, Cy5, and Cy7 and are marketed by Amersham (Piscataway, NJ). Other families of dyes that can be used in the invention include, e.g., polyhalofluorescein-family dyes, hexachlorofluorescein-family dyes, coumarin-family dyes, oxazine-family dyes, thiazine-family dyes, squaraine-family dyes, chelated lanthanide-family dyes, ALEXA FLUOR^{®} dyes (Molecular Probes, Inc., Eugene OR),and BODIPY^{®}-family dyes (Molecular Probes, Inc.)

In addition to fluorescent label(s), other embodiments may comprise probes having one or more quencher moieties. A quencher refers to a chemical moiety that absorbs energy emitted from a fluorescent dye, or otherwise interferes with the ability of the fluorescent dye to emit light. A quencher may re-emit the energy absorbed from a fluorescent dye in a signal characteristic for that quencher, thus a quencher can also be a label. Alternatively, a quencher may dissipate the energy absorbed from a fluorescent dye as heat. Molecules commonly used in FRET include, for example, fluorescein, FAM, JOE, rhodamine, R6G, TAMRA, ROX, DABCYL, and EDANS. Exemplary non-fluorescent quenchers that dissipate energy absorbed from a fluorescent dye include the Black Hole Quenchers™ marketed by Biosearch Technologies, Inc. (Novato, Calif.), Eclipse Dark Quenchers from Epoch Biosciences (Bothell, WA), and Iowa Black (Integrated DNA Technologies, Coralville, IA).

The labels and/or quenchers can be attached to the oligonucleotide probe directly or indirectly by a variety of techniques. Depending on the precise type of label used, the label might be located at the 5' or 3'-end of the probe, or be located internally in the nucleotide sequence. The labels and/or quenchers may be attached directly to a nucleotide, or may be attached indirectly via linkers or spacer arms of various sizes and compositions to facilitate signal interactions. Using commercially available phosphoramidite reagents, one can produce oligomer probes containing functional groups (e.g., thiols or primary amines) at either terminus via an appropriately protected phosphoramidite, and can label them using protocols described in, for example, PCR Protocols: A Guide to Methods and Applications, ed. by Innis et al., Academic Press, Inc., 1990. It is also possible to attach a detectable moiety at the 3'-terminus of the probe by employing, for example, polynucleotide terminal transferase to add a desired moiety, such as, for example, cordycepin ³⁵S-dATP, and biotinylated dUTP.

Methods for introducing oligonucleotide functionalizing reagents to introduce one or more sulfhydryl, amino or hydroxyl moieties into the oligonucleotide probe sequence, typically at the 5'-terminus are described in U.S. Pat. No. 4,914,210. A radioactive (e.g., ³²P-labeled) phosphate group can be introduced at a 5'-terminus of an oligonucleotide by using polynucleotide kinase and [gamma-³²P]ATP. Biotin can be added to the 5'-end by reacting an aminothymidine residue or alkylamino linker, introduced during synthesis, with an N-hydroxysuccinimide ester of biotin. Labels at the 3'-terminus of an oligonucleotide may be added using polynucleotide terminal transferase to add the desired moiety, such as for example, cordycepin ³⁵S-dATP, and biotinylated dUTP. Certain nucleotide derivatives may also be used as labels. For example, etheno-dA and etheno-A are known fluorescent adenine nucleotides that can be incorporated into an oligonucleotide probe. Similarly, etheno-dC is another analog that could be used in probe synthesis.

In certain embodiments of the invention, a probe is multiply-labeled, with each label individually attached to different locations of the probe. In another embodiment of the invention, a single probe is dual-labeled with a fluorescent dye (i.e., a label) and a quencher. When the probe is intact, the fluorescence of the label is quenched by the quencher. Cleaving the probe between the label and quencher results in less quenching of the label's emitted fluorescence. An exemplary combination for this aspect of the invention is the fluorescent dye FAM and the quencher BHQ-2.

### HCV GENOTYPING PROBES

The invention provides compositions for HCV genotyping, where the compositions include at least one HCV genotyping probe that can make an HCV genotype assignment. For example, the invention provides an HCV genotyping probe, *e.g.,* the HCV genotyping probe HCGT27P5'3' of SEQ ID NO:3, that is able to differentiate a large number of HCV genotypes based on Tm discrimination, as illustrated in the Example. This probe has the sequence:
EFFGGAALLGFFAGGAFGAFFGGGTCCTJ (SEQ ID NO: 3) where E = BHQ2; J = cx-FAM; F = propynyl dU; L = propynyl dC.

It will be apparent to one of skill in the art that this probe sequence can be readily modified to obtain substantially functionally equivalent probe molecules, *i.e.,* functionally equivalent molecules that are also able to discriminate multiple HCV genotypes. For example, the probe provided in SEQ ID NO: 3 comprises various labels and/or quenchers (e.g., BHQ2 and cx-FAM). It will be immediately apparent to one of skill in the art that these moieties can be substituted for other types of labels or label systems, and where the probe will retain its critical property of differentially binding a multitude of HCV genotypes and not depart from the essential feature of the invention. Indeed, such labels can even be removed, and the probe still retains its essential property of HCV genotype discrimination.

### T_{M} AND HYBRIDIZATION

One of the benefits of the current invention is that melting/annealing curves are created from the same reaction sample as the kinetic PCR curves, e.g., within the same sample container. In various embodiments herein, the temperature profile of the samples under analysis can optionally be manipulated in several ways in order to produce the melting/annealing curves. For example, the heating and/or cooling of the samples in order to construct the melting/annealing curves is typically done over a determined range of temperatures. The specific temperature range is typically set based upon, e.g., the specific targets/probes under consideration, their sequences (as much as is known at least), the length of the probe(s), etc. In addition to the levels of temperatures in the current invention, the speed of temperature change is also optionally variable in different embodiments. The changes in temperature are optionally continuous, but in some embodiments can be discontinuous.

In interpreting the resulting Tm curve in relation to determining the underlying target genotype, sequence, etc., reference is optionally made between the Tm curve of the target sample(s) and one or more control Tm curves or between the Tm curve of the samples and the predicted Tm curve that would be expected given the probe sequence and what is known or assumed to be the target sequence, the reaction conditions, etc. Various embodiments herein can optionally use one or both of such means to interpret any Tm curves to determine genotype, sequence, identity, etc. of a target nucleic acid in a sample.

### EXEMPLARY USES OF THE INVENTION

The current invention is optionally utilized in diagnostics, e.g., of medical conditions. Such conditions can include diagnostics involving infectious diseases, as well as noninfectious medical conditions such as cancer, etc. In determination of a causative agent in a disease state in a subject, the current invention can distinguish between causative agents for disease in situations wherein many diverse agents could possibly cause the disease. For example, upper respiratory infections (URI) are very dangerous for those infected with them and correct diagnosis of the underlying disease agent is very important for proper treatment. Whether the URI is due to a virus, bacteria, etc. greatly influences the proper course of patient treatment. In addition, gram positive and gram negative bacterial infections require different courses of treatment. With properly selected primers and probes, the current invention can determine the presence or absence of particular agents within a sample, as well as distinguish between certain agents present in a sample.

Other diagnostic uses of the current invention involve differentiation between causative agents that are close to one another in sequence. For example, as illustrated in the Example herein, HCV subtypes can be distinguished one from another through use of the current invention. Through use of the proper probes and primers, a host of other related disease agents can be differentiated and subjects having such disease agents can be treated appropriately based upon such differentiation. For example, HIV substrains, HCV strains/substrains, flavivirus types and the like can all optionally be distinguished by embodiments of the current invention.

In other embodiments, the current invention can optionally be used for identification of nucleic acids in relation to allele typing for higher organisms (e.g., as in genetic screening), detection of certain cancers, HLA typing, etc.

### KITS, SYSTEMS AND REACTION MIXTURES

Also disclosed are kits for the detection and/or quantification and/or identification of nucleic acids. Such kits can comprise any combination of, e.g., appropriate 5'-nuclease and hybridization probes (e.g., based upon the specific nucleic acids to be tested, genotyped, etc.), appropriate primers (e.g., to amplify the target nucleic acid), reagents and materials for the amplification and identification of the target nucleic acid such as buffers, nucleotides, salts, etc. The kit optionally further comprises an instruction set or user manual detailing preferred methods of using the kit components for discovery or application of diagnostic sets. Typically, the kit contains, in addition to the above components, additional materials which can include, e.g., instructions for performing the methods of the invention for detection and/or quantification and/or identification of nucleic acids, packaging material, and one or more containers.

Also disclosed is a system for the detection and/or quantification and/or identification of nucleic acids. Such systems can comprise, e.g., one or more fluorescently labeled hybridization probes; one or more labeled 5'-nuclease probes (which can be the same as the hybridization probes); two or more kinetic PCR primers that are specific for amplification of nucleic acid targets and which are present in unequal amounts for asymmetric kinetic PCR; one or more containers for the probes, primers, and other PCR constituents; one or more thermal modulator that is operably connected (i.e., thermally connected) to the container and that can controllably change the temperature in the container; a detector configured to detect the fluorescent signals from the various probes in the reactions (e.g., whether hybridization probes, 5'-nuclease probes); a monitor for visually displaying the data; and a controller that is operably connected to the monitor, the detector and/or and the thermal modulator and which can include instruction sets for controlling the thermal modulator and the monitor, the detector and/or and the thermal modulator, as well as instruction sets for correlating the fluorescent signals and the temperature in the container with the presence of one or more target nucleic acid. If the probes are labeled through nonfluorescent means (e.g., radioactive), the detectors in such systems comprise those that detect such nonfluorescent activity.

Also disclosed are reaction mixtures having primers specific for amplification of at least one nucleic acid target, one or more labeled 5'-nuclease probes and one or more labeled hybridization probes wherein the 5'-nuclease probes and the hybridization probes can be either the same probe or different probes. Herein, the primers are present in different amounts and the hybridization probes are present in a greater amount than the amount of the limiting primer (i.e., the primer present in the smaller amount).

Kits, etc., disclosed herein can include those done in a single tube or container, which allows the reactions to be done without opening of the tube/container.

### EXAMPLE

The following example is offered to illustrate the claimed invention. One of skill will recognize a variety of non-critical parameters that may be altered. It is understood that the examples and embodiments described herein are for illustrative purposes only and that various modifications or changes in light thereof will be suggested to persons skilled in the art.

### EXAMPLE

### Use of the Invention to Determine HCV Genotype

Determining the genotype of an HCV (hepatitis C virus) positive sample has important clinical significance and utility. Knowing the genotype of the specific virus allows physicians to select the correct treatment regimen. As explained above, the current invention can be used to discriminate amongst a number of nucleic acid targets (e.g., even ones that are closely related in sequence) such as HCV genotypes. The methods of the invention use an oligonucleotide probe that is specifically designed to be at least partially complementary to a region of target sequence divergence (e.g., a region that shows divergence between different strains, alleles, species, etc.) in conjunction with using the different Tms obtained from post-PCR melting and/or annealing analysis to distinguish the nucleic acid targets.

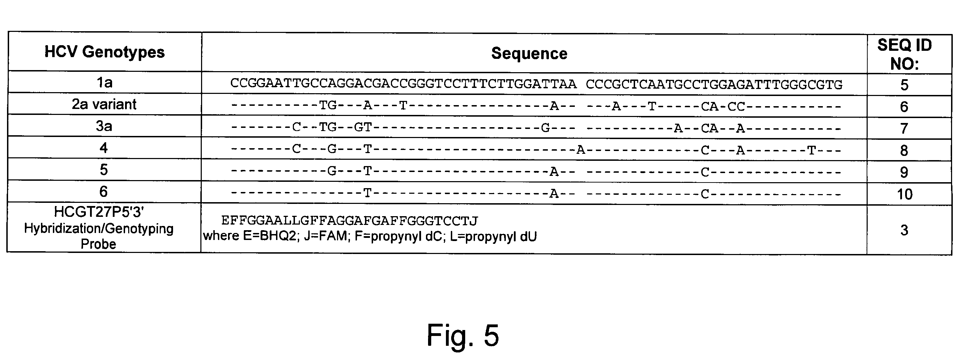

**Figure 5** shows aligned sequences of 6 different HCV genotypes (namely types 1a, 2a, 3a, 4, 5, and 6) along with an exemplary hybridization probe capable of use in the current invention to discriminate between the different HCV genotypes.

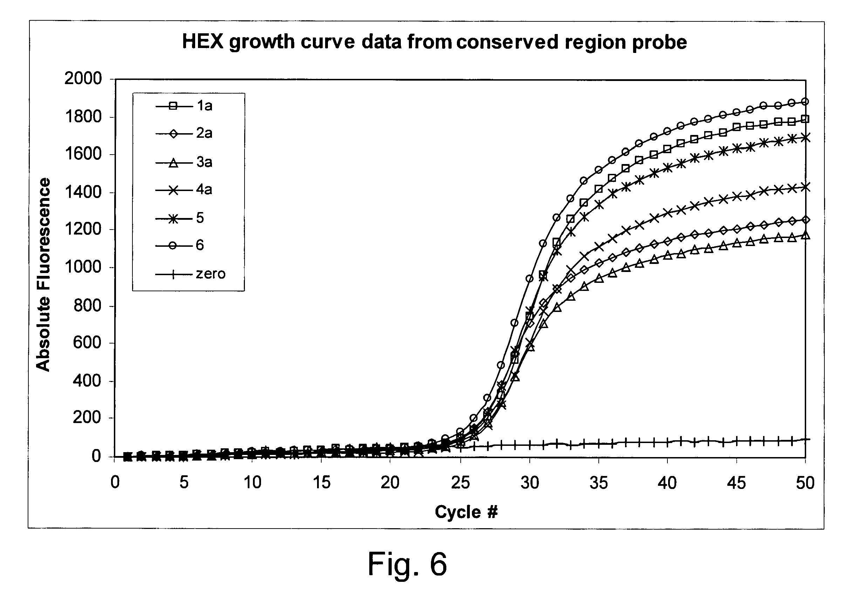

As indicated previously, the 5'-nuclease probe(s) can optionally use a different color-channel than the hybridization probe(s). For example, as seen in **Figures 6-8**, a FAM-labeled hybridization probe (to distinguish between different genotypes of HCV) and a HEX-labeled 5'-nuclease probe were utilized with selected RNA transcripts of HCV genotypes 1a, 2a, 3a, 4, 5, and 6.

In the current example, the asymmetric PCR sample master mix (i.e., the PCR constituents) consisted of: 2.5% glycerol; 5% DMSO; 50 mM Tricine, pH 8.3; 90 mM potassium acetate; 300 µM dATP, 300 µM dGTP, 300 µM dCTP, 550 µM dUTP; 0.1 µM upstream (limiting) primer; 0.5 µM downstream (excess) primer; 0.2 µM hydrolysis probe, 0.2 µM hybridization probe; 10U uracil-N-glycosylase; 40 U ZO5 DNA polymerase; and 2.7 mM manganese acetate.

Template RNA for generating HCV amplicons by RT-PCR was derived by in vitro transcription from plasmids carrying HCV genomic material inserts corresponding to types 1a, 2a, 3a, 4, 5 and 6. The sequences of these inserts correspond to the consensus sequences of each of the respective types as described in Figure 5. Following the *in vitro* transcription, the RNA was purified by oligo-dT-sepharose chromatography.

In the example, the hybridization/genotyping probe was present at twice the concentration of the limiting primer to ensure that not all the probe was cleaved. The excess primer was present at 5x the limiting primer concentration to ensure an excess of single-stranded amplicon for the hybridization probe to bind to. The thermocycling profile used for the example was: 50°C for 5 minutes (UNG step); 59°C for 30 minutes (RT step); 94°C for 20 seconds - 58°C for 40 seconds x 60 cycles; 94°C for 60 seconds; and 40°C to 90°C in 1°C steps - melt step. The oligonucleotides included in the reaction were as follows:

| Name | Sequence | SEQ ID NO: |
|---|---|---|
| upstream primer | GCAGAAAGCGTCTAGCCATGGCGTTE where E = t-butyl benzyl dA | 1 |
| downstream primer (the RT primer) | GCAAGCACCCTATCAGGCAGTACCACAE | 2 |
| | where E = t-butyl benzyl dA | |
| HCGT27P5'3' | EFFGGAALLGFFAGGAFGAFFGGGTCCTJ | 3 |
| hybridization/genotyping probe | where E = BHQ2; J = cx-FAM; F = propynyl dU; L = propynyl dC | |
| hydrolysis probe | ECTCACCGGTJCCGCAGACCACTATGGCTCTCCCP | 4 |
| (i.e., 5'-nuclease probe) | where E = CY5; J = HEX; P = phosphate | |

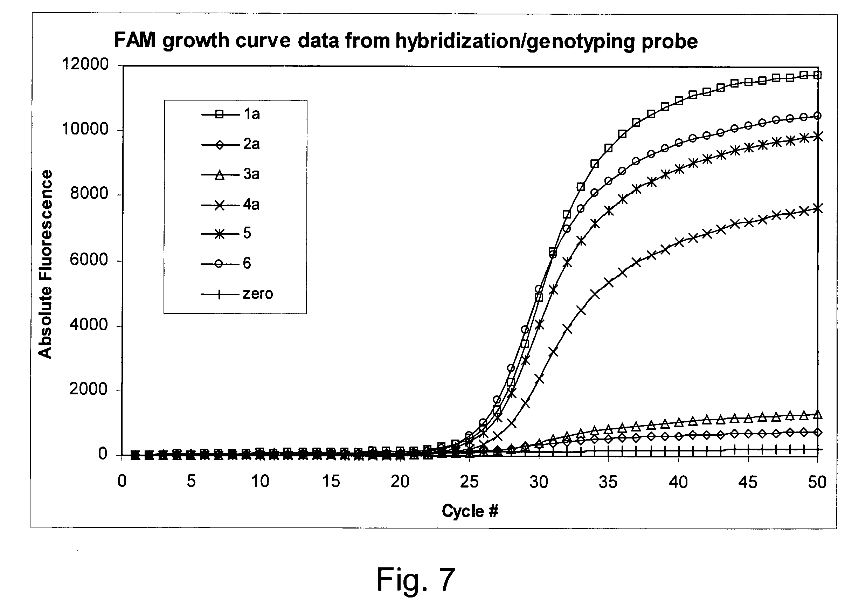

The growth curve data from the HEX-labeled 5'-nuclease probe is shown in Figure 6. In certain embodiments, such probe is a conserved probe which has no mismatches between the probe and the selected nucleic acid transcripts used and should therefore detect all genotypes equally. The growth curve data from the FAM-labeled hybridization probe, however, as shown in Figure 7, arise from the probe which does not show complete complementarity to all transcripts tested and therefore shows much more variability. The reaction with subtypes 2a and 3a, which have the greatest amount of mismatch between the probe and the transcripts, generate very little 5'-nuclease signal.

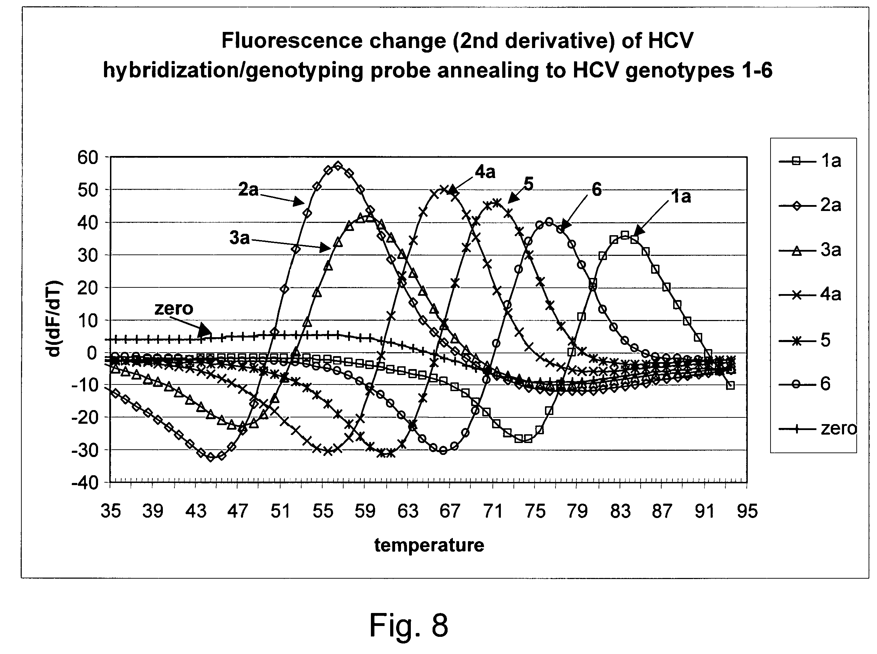

Figure 8 shows post-PCR annealing data for each transcript and the hybridization/genotyping probe. In the current example, the melting/annealing curves were produced on an ABI Prism 7700 Thermocycler. Those of skill in the art will be quite familiar with basic protocols and associated parameters (e.g., control of stringency, thermocycler, etc.) for construction of thermal melting/annealing curves between nucleic acids. As can be seen, a wide range of Tms exist between the different HCV genotypes, e.g., between 50°C and 78°C. As explained previously, the higher Tms arise from matches between the hybridization probe and transcripts from HCV genotypes that are closely matched in sequence, while those genotypes that have less sequence match with the probe in such region show lower Tm. The range of different Tms seen in Figure 8 allows for easy differentiation between the different genotypes. While the Tms for genotype 2a and genotype 3a are closer to each other than the other readings, selection of a different genotyping probe (e.g., different in sequence, but binding to the same region or binding to a different region on the transcripts) could optionally be used to further clarify the difference between the genotypes in the sample. Also, changes in reaction conditions (e.g., temperature, salt concentrations, etc.) could also optionally be used to help differentiate such. Figure 8 illustrates that, as with all melting/annealing based assays, performance is greatly affected by new or unknown mismatches in the target region. Different embodiments herein can optionally account for such new or unknown mismatches in probe binding regions by constructing a variety of hybridization probes, etc.

While the foregoing invention has been described in some detail for purposes of clarity and understanding, it will be clear to one skilled in the art from a reading of this disclosure that various changes in form and detail can be made. For example, all the techniques and apparatus described above may be used in various combinations.

## Claims

1. A method for classifying, identifying, quantifying and/or genotyping one or more nucleic acid targets in a sample, the method comprising:
a) performing an asymmetric kinetic PCR in a reaction mixture containing one or more labeled 5'- nuclease probes and one or more labeled hybridization probes wherein said one or more 5'-nuclease probes and said one or more hybridization probes can be the same probe(s) or different probes in sequence ;
b) monitoring fluorescent signals generated from the one or more labeled 5'-nuclease probes to create one or more growth curves from the kinetic PCR for calculating a Cₜ (cycle threshold) value and thereby determining the copy number, the genotype or the target identity;
c) modifying the temperature of the reaction mixture after the kinetic PCR to cause a change in association between the one or more labeled hybridization probes and the one or more nucleic acid targets;
d) monitoring one or more fluorescent signals generated from the one or more labeled hybridization probes bound to the excess strand generated in the asymmetric kinetic PCR thereby producing a melting curve or annealing curve;
e) correlating the melting curve or annealing curve of step d) to a melting or annealing curve of a completely complementary probe of one or more known nucleic acid targets, thus, identifying the one or more nucleic acid targets in the sample,
wherein the asymmetric kinetic PCR comprises a first primer and at least a second primer, wherein the amount of the first primer is greater than the amount of the second primer and wherein the hybridization probe is present in greater amount than the second primer.

2. The method of claim 1, wherein identifying the one or more nucleic acid targets comprises identifying one or more organisms or organism strains having said nucleic acids.

3. The method of claim 1, wherein the asymmetric kinetic PCR comprises a first primer and at least a second primer, and wherein the PCR comprises at least a 2:1 ratio of first primer to second primer.

4. The method of claim 1, wherein the asymmetric kinetic PCR comprises a first primer and at least a second primer, and wherein the PCR comprises at least a 3:1 ratio of first primer to second primer.

5. The method of claim 1, wherein the asymmetric kinetic PCR comprises a first primer and at least a second primer, and wherein the PCR comprises at least a 4:1 ratio of first primer to second primer.

6. The method of claim 1, wherein the asymmetric kinetic PCR comprises a first primer and at least a second primer, and wherein the PCR comprises at least a 5:1 ratio of first primer to second primer.

7. The method of claim 1, wherein the sequence of the one or more labeled 5'-nuclease probes is the same as the sequence of the one or more labeled hybridization probes.

8. The method of claim 1, wherein the one or more labeled 5'-nuclease probes are different from the one or more labeled hybridization probes.

9. The method of claim 1, wherein at least one of the one or more hybridization probes is completely complementary to at least one region of at least one nucleic acid target.

10. The method of claim 1, wherein at least one of the one or more hybridization probes is partially complementary to at least one region of at least one nucleic acid target.

11. The method of claim 1, wherein the asymmetric kinetic PCR is monitored by a first fluorescence and wherein monitoring of the change in association of the hybridization probes is monitored by a second fluorescence which second fluorescence is different from said first fluorescence.

12. The method of claim 1, wherein the asymmetric kinetic PCR is monitored by a first fluorescence and wherein monitoring of the change in association of the hybridization probes is monitored by a second fluorescence which second fluorescence is the same as said first fluorescence.

13. The method of claim 1, wherein the one or more nucleic acid targets comprise hepatitis C virus (HCV) nucleic acid.

14. The method of claim 17, wherein identifying the one or more HCV nucleic acid target identifies one or more HCV strain in the sample.

15. The method of claim 1, wherein at least one of the hybridization probes is substantially complementary to an HCV strain genotype.

16. The method of claim 1, wherein the reaction mixture comprises a first hybridization probe and at least a second hybridization probe, wherein the first hybridization probe is substantially complementary to a first HCV strain genotype and the at least a second hybridization probe is substantially complementary to a second HCV strain genotype.

17. The method of claim 1, wherein the hybridization probe comprises a polynucleotide sequence of SEQ ID NO: 3.

## Patentansprüche

1. Verfahren zur Klassifizierung, Identifizierung, Quantifizierung und/oder Genotypisierung eines oder mehrerer Nukleinsäureziele in einer Probe, wobei das Verfahren Folgendes umfasst:
a) Durchführen einer Asymmetrische-Kinetik-PCR in einem Reaktionsgemisch, das eine oder mehrere 5'-Nuklease-Sonden und eine oder mehrere Hybridisierungssonden enthält, wobei die eine oder mehreren 5'-Nuklease-Sonden und die eine oder mehreren Hybridisierungssonden die gleiche(n) Sonde(n) oder Sonden mit unterschiedlicher Sequenz sein können;
b) Beobachten von Fluoreszenzsignalen, die von der einen bzw. den mehreren 5'-Nuklease-Sonde(n) erzeugt werden, so dass eine oder mehrere Wachstumskurven aus der Kinetik-PCR zur Berechnung eines Cₜ-Werts (Zyklus-Schwellenwerts) entstehen, wodurch die Kopienzahl, der Genotyp oder die Zielidentität bestimmt wird;
c) Modifizieren der Temperatur des Reaktionsgemischs nach der Kinetik-PCR, um eine Änderung der Assoziation zwischen der einen bzw. den mehreren markierten Hybridisierungssonde(n) und dem einen bzw. den mehreren Nukleinsäureziel(en) herbeizuführen;
d) Beobachten eines oder mehrerer Fluoreszenzsignale, die von der einen bzw. den mehreren markierten Hybridisierungssonde(n), die an den in der Asymmetrische-Kinetik-PCR erzeugten überschüssigen Strang gebunden ist bzw. sind, erzeugt werden, wodurch eine Schmelzkurve oder eine Annealing-Kurve produziert wird;
e) Korrelieren der Schmelzkurve oder Annealing-Kurve aus Schritt d) mit einer Schmelz- bzw. Annealing-Kurve einer vollständig komplementären Sonde eines oder mehrerer bekannter Nukleinsäureziele, womit das eine bzw. die mehreren Nukleinsäureziel(e) in der Probe identifiziert werden,
wobei die Asymmetrische-Kinetik-PCR einen ersten Primer und wenigstens einen zweiten Primer umfasst, wobei die Menge des ersten Primers größer als die Menge des zweiten Primers ist und wobei die Hybridisierungssonde in größerer Menge vorliegt als der zweite Primer.

2. Verfahren nach Anspruch 1, wobei Identifizieren des einen bzw. der mehreren Nukleinsäureziel(s/e) Identifizieren eines oder mehrerer Organismen oder Organismusstämme, die die Nukleinsäuren aufweisen, umfasst.

3. Verfahren nach Anspruch 1, wobei die Asymmetrische-Kinetik-PCR einen ersten Primer und wenigstens einen zweiten Primer umfasst und wobei die PCR wenigstens ein 2:1-Verhältnis von erstem Primer zu zweitem Primer umfasst.

4. Verfahren nach Anspruch 1, wobei die Asymmetrische-Kinetik-PCR einen ersten Primer und wenigstens einen zweiten Primer umfasst und wobei die PCR wenigstens ein 3:1-Verhältnis von erstem Primer zu zweitem Primer umfasst.

5. Verfahren nach Anspruch 1, wobei die Asymmetrische-Kinetik-PCR einen ersten Primer und wenigstens einen zweiten Primer umfasst und wobei die PCR wenigstens ein 4:1-Verhältnis von erstem Primer zu zweitem Primer umfasst.

6. Verfahren nach Anspruch 1, wobei die Asymmetrische-Kinetik-PCR einen ersten Primer und wenigstens einen zweiten Primer umfasst und wobei die PCR wenigstens ein 5:1-Verhältnis von erstem Primer zu zweitem Primer umfasst.

7. Verfahren nach Anspruch 1, wobei die Sequenz der einen oder mehreren markierten 5'-Nuklease-Sonden die gleiche wie die Sequenz der einen oder mehreren markierten Hybridisierungssonden ist.

8. Verfahren nach Anspruch 1, wobei die eine bzw. die mehreren markierte(n) 5'-Nuklease-Sonde(n) von der einen bzw. den mehreren markierten Hybridisierungssonde(n) verschieden ist bzw. sind.

9. Verfahren nach Anspruch 1, wobei wenigstens eine der einen oder mehreren markierten Hybridisierungssonden vollständig komplementär zu wenigstens einem Bereich wenigstens eines Nukleinsäureziels ist.

10. Verfahren nach Anspruch 1, wobei wenigstens eine der einen oder mehreren markierten Hybridisierungssonden teilweise komplementär zu wenigstens einem Bereich wenigstens eines Nukleinsäureziels ist.

11. Verfahren nach Anspruch 1, wobei die Asymmetrische-Kinetik-PCR über eine erste Fluoreszenz beobachtet wird und wobei das Beobachten der Änderung der Assoziation der Hybridisierungssonden über eine zweite Fluoreszenz beobachtet wird, wobei die zweite Fluoreszenz von der ersten Fluoreszenz verschieden ist.

12. Verfahren nach Anspruch 1, wobei die Asymmetrische-Kinetik-PCR über eine erste Fluoreszenz beobachtet wird und wobei das Beobachten der Änderung der Assoziation der Hybridisierungssonden über eine zweite Fluoreszenz beobachtet wird, wobei die zweite Fluoreszenz die gleiche wie die erste Fluoreszenz ist.

13. Verfahren nach Anspruch 1, wobei das eine bzw. die mehreren Nukleinsäureziel(e) Hepatitis-C-Virus(HCV)-Nukleinsäure umfasst bzw. umfassen.

14. Verfahren nach Anspruch 17, wobei durch Identifizieren des einen bzw. der mehreren Nukleinsäureziel(s/e) ein oder mehrere HCV-Stämme in der Probe identifiziert werden.

15. Verfahren nach Anspruch 1, wobei wenigstens eine der Hybridisierungssonden weitgehend komplementär zu einem HCV-Stamm-Genotyp ist.

16. Verfahren nach Anspruch 1, wobei das Reaktionsgemisch eine erste Hybridisierungssonde und wenigstens eine zweite Hybridisierungssonde umfasst, wobei die erste Hybridisierungssonde weitgehend komplementär zu einem ersten HCV-Stamm-Genotyp ist und die wenigstens eine zweite Hybridisierungssonde weitgehend komplementär zu einem zweiten HCV-Stamm-Genotyp ist.

17. Verfahren nach Anspruch 1, wobei die Hybridisierungssonde eine Polynukleotidsequenz unter SEQ ID NO: 3 umfasst.

## Revendications

1. Procédé pour la classification, l'identification, la quantification et/ou le génotypage d'un ou plusieurs acides nucléiques cibles dans un échantillon, le procédé comprenant :
a) la réalisation d'une PCR cinétique asymétrique dans un mélange réactionnel contenant une ou plusieurs sondes 5'-nucléases marquées et une ou plusieurs sondes d'hybridation marquées, dans laquelle lesdites une ou plusieurs sondes 5'-nucléases et lesdites une ou plusieurs sondes d'hybridation peuvent être la(les) même(s) sonde(s) ou des sondes différentes en termes de séquence ;
b) la surveillance des signaux fluorescents générés par la ou les sondes 5'-nucléases marquées afin de créer une ou plusieurs courbes de croissance à partir de la PCR cinétique pour le calcul d'une valeur Cₜ (seuil de cycle) et déterminer ainsi le nombre de copies, le génotype ou l'identité de la cible ;
c) la modification de la température du mélange réactionnel après la PCR cinétique afin de provoquer un changement dans l'association entre la ou les sondes d'hybridation marquées et le ou les acides nucléiques cibles ;
d) la surveillance d'un ou de plusieurs signaux de fluorescence générés à partir de la ou des sondes d'hybridation marquées liées au brin excédentaire généré dans la PCR cinétique asymétrique produisant ainsi une courbe de fusion ou une courbe d'annelage ;
e) la corrélation de la courbe de fusion ou de la courbe d'annelage de l'étape d) à une courbe de fusion ou d'annelage d'une sonde totalement complémentaire d'un ou de plusieurs acides nucléiques cibles connus, identifiant ainsi, le ou les acides nucléiques cibles dans l'échantillon,
dans lequel la PCR cinétique asymétrique comprend une première amorce et au moins une deuxième amorce, dans lequel la quantité de la première amorce est supérieure à la quantité de la deuxième amorce, et dans lequel la sonde d'hybridation est présente en plus grande quantité que la deuxième amorce.

2. Procédé selon la revendication 1, dans lequel l'identification du ou des acides nucléiques cibles comprend l'identification d'un ou de plusieurs organismes ou souches d'organisme ayant lesdits acides nucléiques.

3. Procédé selon la revendication 1, dans lequel la PCR cinétique asymétrique comprend une première amorce et au moins une deuxième amorce, et dans lequel la PCR comprend au moins un rapport de la première amorce à la deuxième amorce de 2:1.

4. Procédé selon la revendication 1, dans lequel la PCR cinétique asymétrique comprend une première amorce et au moins une deuxième amorce, et dans lequel la PCR comprend au moins un rapport de la première amorce à la deuxième amorce de 3:1.

5. Procédé selon la revendication 1, dans lequel la PCR cinétique asymétrique comprend une première amorce et au moins une deuxième amorce, et dans lequel la PCR comprend au moins un rapport de la première amorce à la deuxième amorce de 4:1.

6. Procédé selon la revendication 1, dans lequel la PCR cinétique asymétrique comprend une première amorce et au moins une deuxième amorce, et dans lequel la PCR comprend au moins un rapport de la première amorce à la deuxième amorce de 5:1.

7. Procédé selon la revendication 1, dans lequel la séquence de la ou des sondes 5'-nucléases marquées est identique à la séquence de la ou des sondes d'hybridation marquées.

8. Procédé selon la revendication 1, dans lequel la ou les sondes 5'-nucléases marquées sont différentes de la ou des sondes d'hybridation marquées.

9. Procédé selon la revendication 1, dans lequel au moins l'une de la ou des sondes d'hybridation est totalement complémentaire d'au moins une région d'au moins un acide nucléique cible.

10. Procédé selon la revendication 1, dans lequel au moins l'une de la ou des sondes d'hybridation est partiellement complémentaire d'au moins une région d'au moins un acide nucléique cible.

11. Procédé selon la revendication 1, dans lequel la PCR cinétique asymétrique est surveillée par une première fluorescence et dans lequel la surveillance du changement dans l'association des sondes d'hybridation est surveillée par une deuxième fluorescence, deuxième fluorescence qui est différente de ladite première fluorescence.

12. Procédé selon la revendication 1, dans lequel la PCR cinétique asymétrique est surveillée par une première fluorescence et dans lequel la surveillance du changement dans l'association des sondes d'hybridation est surveillée par une deuxième fluorescence, deuxième fluorescence qui est la même que ladite première fluorescence.

13. Procédé selon la revendication 1, dans lequel le ou les acides nucléiques cibles comprennent un acide nucléique de virus de l'hépatite C (VHC).

14. Procédé selon la revendication 17, dans lequel l'identification du ou des acides nucléiques cibles de VHC identifie une ou plusieurs souches de VHC dans l'échantillon.

15. Procédé selon la revendication 1, dans lequel au moins l'une des sondes d'hybridation est sensiblement complémentaire d'un génotype de souche de VHC.

16. Procédé selon la revendication 1, dans lequel le mélange réactionnel comprend une première sonde d'hybridation, et au moins une deuxième sonde d'hybridation, dans lequel la première sonde d'hybridation est sensiblement complémentaire d'un premier génotype de souche de VHC et, la ou les deuxième sondes d'hybridation sont sensiblement complémentaires d'un deuxième génotype de souche de VHC.

17. Procédé selon la revendication 1, dans lequel la sonde d'hybridation comprend une séquence polynucléotidique de SEQ ID NO: 3.
